# EUROPÄISCHE PATENTSCHRIFT

(11) **EP 1 682 477 B1**
(45) Veröffentlichungstag und Bekanntmachung des Hinweises auf die Patenterteilung: **13.04.2016**
(21) Anmeldenummer: 04765891.9
(22) Anmeldetag: 08.10.2004
(51) Int. Cl.: C07C 51/25, C07C 57/04

(54) **VERFAHREN ZUM LANGZEITBETRIEB EINER HETEROGEN KATALYSIERTEN GASPHASENPARTIALOXIDATION VON ACROLEIN ZU ACRYLSÄURE**
METHOD FOR LONG TERM OPERATION OF A HETEROGENEOUSLY CATALYSED GAS PHASE PARTIAL OXIDATION OF ACROLEIN IN ORDER TO FORM ACRYLIC ACID
PROCEDE D'UTILISATION A LONG TERME D'UNE OXYDATION PARTIELLE EN PHASE GAZEUSE D'ACROLEINE EN ACIDE ACRYLIQUE CATALYSEE DE FA ON HETEROGENE

(30) Priorität: 29.10.2003 DE 10350822; 29.10.2003 US 514918 P
(43) Veröffentlichungstag der Anmeldung: 26.07.2006
(73) Patentinhaber: BASF SE, 67056 Ludwigshafen am Rhein (DE)
(72) Erfinder: DIETERLE, Martin, 68167 Mannheim (DE); PETZOLDT, Jochen, 67273 Weisenheim am Berg (DE); MÜLLER-ENGEL, Klaus Joachim, 76297 Stutensee (DE)
(74) Vertreter: BASF IP Association
(86) Internationale Anmeldenummer: PCT/EP2004/011259
(87) Internationale Veröffentlichungsnummer: WO 2005/047226

(56) Entgegenhaltungen:
- EP-A- 0 614 872
- US-B1- 6 346 646

## Beschreibung

Vorliegende Erfindung betrifft ein Verfahren zum Langzeitbetrieb einer heterogen katalysierten Gasphasenpartialoxidation von Acrolein zu Acrylsäure, bei dem man ein Acrolein, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Mo und V enthält, und bei dem man, um der Deaktivierung des Katalysatorfestbetts entgegenzuwirken, die Temperatur des Katalysatorfestbetts über die Zeit erhöht.

Acrylsäure bildet ein reaktives Monomer, das als solches oder in Form seiner Alkylester z.B. zur Herstellung von Polymerisaten, die u.a. als Klebstoffe oder Wasser absorbierende Materialien Verwendung finden können, geeignet ist.

Es ist bekannt, Acrylsäure großtechnisch durch ein Verfahren der heterogen katalysierte Gasphasenpartialoxidation von Acrolein zu Acrylsäure, bei dem man ein Acrolein, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Mo und V enthält, herzustellen (vgl. z.B. DE-A 4431949, WO 00/53559).

Es ist auch bekannt, dass ein solches Verfahren der heterogen katalysierten Gasphasenpartialoxidation von Acrolein zu Acrlysäure an ein und demselben Katalysatorfestbett im wesentlichen kontinuierlich über längere Zeiträume betrieben werden kann. Allerdings verliert das Katalysatorfestbett dabei im Verlauf der Betriebszeit an Qualität. In der Regel verschlechtern sich sowohl seine Aktivität als auch die Selektivität der Zielproduktbildung.

Um das Katalysatorfestbett, dessen Fertigung und Austausch vergleichsweise aufwendig und kostspielig sind, trotzdem möglichst lange in einem damit beschickten Reaktor betreiben zu können, wird im Stand der Technik auf unterschiedlichste Art und Weise versucht, dem Alterungsprozess des Katalysatorfestbetts entgegenzuwirken.

Die EP-A 990 636 (z.B. Seite 8, Zeilen 13 bis 15) und die EP-A 1 106 598 (z.B. Seite 13, Zeilen 43 bis 45) schlagen vor, die Minderung der Qualität des Katalysatorfestbetts dadurch weitgehend zu kompensieren, dass im Verlauf der Betriebszeit, unter ansonsten weitgehend gleichbleibenden Betriebsbedingungen, die Temperatur des Katalysatorfestbetts nach und nach erhöht wird, um den Acroleinumsatz bei einmaligem Durchgang des Reaktionsgasgemischs durch das Katalysatorfestbett im wesentlichen beizubehalten.

Unter der Temperatur des Katalysatorfestbetts wird dabei die Temperatur des Katalysatorfestbetts bei Ausübung des Partialoxidationsverfahrens jedoch in fiktiver Abwesenheit einer chemischen Reaktion (d.h., ohne den Einfluss der Reaktionswärme) verstanden. Dies soll auch in dieser Schrift gelten. Unter effektiver Temperatur des Katalysatorfestbetts soll in dieser Schrift dagegen die tatsächliche Temperatur des Katalysatorfestbetts unter Einbezug der Reaktionswärme der Partialoxidation verstanden werden. Ist die Temperatur des Katalysatorfestbetts längs des Katalysatorfestbetts nicht konstant (z.B. im Fall von mehreren Temperaturzonen), so meint der Begriff Temperatur des Katalysatorfestbetts in dieser Schrift den (Zahlen)mittelwert der Temperatur entlang des Katalysatorfestbetts.

Von Bedeutung im vorgenannten Zusammenhang ist, dass die Temperatur des Reaktionsgasgemischs (und damit auch die effektive Temperatur des Katalysatorfestbetts) beim Durchgang durch das Katalysatorfestbett einen Höchstwert (den sogenannten Heißpunktwert) durchläuft. Die Differenz zwischen Heißpunktwert und der Temperatur des Katalysatorfestbetts an der Stelle des Heißpunktwertes wird als Heißpunktausdehnung bezeichnet.

Nachteilig an der in der EP-A 990 636 sowie in der EP-A 1 106 598 empfohlenen Verfahrensweise ist, dass sich mit zunehmender Erhöhung der Temperatur des Katalysatorfestbetts sein Alterungsprozess beschleunigt (bestimmte Bewegungsprozesse innerhalb der Katalysatoren, die zur Alterung beitragen, laufen z.B. schneller ab). Dies in der Regel vor allem auch deshalb, weil die Heißpunktausdehnung mit einer Erhöhung der Temperatur des Katalysatorfestbetts meist noch stärker zunimmt als die Temperatur des Katalysatorfestbetts selbst (vgl. z.B. Seite 12, Zeilen 45 bis 48 der EP-A 1 106 598 und Seite 8, Zeilen 11 bis 15 der EP-A 990 636). Die effektive Temperatur des Katalysatorfestbetts nimmt im Heißpunktbereich daher meist überproportional zu, was die Alterung des Katalysatorfestbetts zusätzlich fördert.

Bei Erreichen eines Höchstwerts der Temperatur des Katalysatorfestbetts wird daher das Katalysatorfestbett üblicherweise vollständig ausgetauscht.

Nachteilig an einem solchen Komplettaustausch ist jedoch, dass er vergleichsweise aufwendig ist. Das Verfahren der Acrylsäureherstellung muss für längere Zeit unterbrochen werden und die Kosten der Katalysatorherstellung sind ebenfalls erheblich.

Erwünscht sind daher Betriebsweisen für Verfahren einer heterogen katalysierten Gasphasenpartialoxidation von Acrolein zu Acrylsäure, die dabei behilflich sind, die Standzeit des Katalysatorfestbetts im Reaktor weitestgehend in die Länge zu ziehen.

Die DE-A 10 232 748 empfiehlt diesbezüglich, anstatt das Katalysatorfestbett vollständig auszutauschen, nur eine Teilmenge desselben durch eine frische Katalysatorbeschickung zu ersetzen.

Nachteilig an diesem Vorschlag ist, dass auch mit einem Teilwechsel des Katalysatorfestbetts bereits ein nennnswerter Aufwand einhergeht.

Die EP-A 614 872 empfiehlt, die Standzeit des Katalysatorfestbetts dadurch zu verlängern, dass man nach mehrjähriger Betriebsdauer des Katalysatorfestbetts, mit der Erhöhungen der Temperatur desselben von 15°C bis 30°C und mehr einhergehen, das Verfahren der Partialoxidation unterbricht, und bei Katalysatorfestbetttemperaturen von 260 bis 450°C durch selbiges ein Gasgemisch aus Sauerstoff, Wasserdampf und Inertgas führt und anschließend die Partialoxidation fortsetzt.

In diesem Zusammenhang sollen in dieser Schrift als Inertgase in einem Gasgemisch, das unter bestimmten Bedingungen durch das Katalysatorfestbett geführt wird, solche Gase verstanden werden, die bei der Durchführung durch das Katalysatorfestbett zu wenigstens 95 mol.-%, bevorzugt zu wenigstens 98 mol.-%, ganz besonders bevorzugt zu wenigstens 99 mol-% oder 99,5 mol-% unverändert erhalten bleiben. Das erfindungsgemäß einzusetzende Gasgemisch G betreffend soll Wasserdampf und CO nicht unter dem Begriff Inertgas subsummieren.

Nachteilig an der Verfahrensweise der EP-A 614 872 ist jedoch, dass bis zum Zeitpunkt der Unterbrechung die Alterung des Katalysatorfestbetts ungebremst fortschreitet und gefördert wird.

Wünschenswert wäre insbesondere ein Verfahren zum Langzeitbetrieb einer heterogen katalysierten Gasphasenpartialoxidation von Acrolein zu Acrylsäure, bei dem der Katalysatoralterung auf eine Art und Weise entgegengewirkt wird, mit der die Ausprägung der Heißpunktausdehnung über die Zeit geringer ist als bei den Verfahren des Standes der Technik.

Demgemäss wurde ein Verfahren zum Langezeitbetrieb einer heterogen katalysierten Gasphasenpartialoxidation von Acrolein zu Acrylsäure, bei dem man ein Acrolein, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Mo und V enthält, und bei dem man, um der Deaktivierung des Katalysatorfestbetts entgegenzuwirken, die Temperatur des Katalysatorsfestbetts über die Zeit erhöht, gefunden, das dadurch gekennzeichnet ist, dass man, bevor die Temperaturerhöhung des Katalysatorfestbetts dauerhaft ≥ 10°C bzw. ≥ 8°C beträgt, die Gasphasenpartialoxidation wenigstens einmal unterbricht, und bei einer Temperatur des Katalysatorfestbetts von 200 bis 450°C (vorzugsweise 250 bis 400°C, oft 300 bis 400°C, oder 250 bis 350°C, oder 250 bis 300°C) ein an Acrolein freies, molekularen Sauerstoff, Inertgas und gegebenenfalls Wasserdampf sowie gegebenenfalls CO enthaltendes, oxidierend wirkendes Gasgemisch G durch das Katalysatorfestbett führt (vorzugsweise besteht das Gasgemisch G im wesentlichen nur oder nur aus den vorgenannten Bestandteilen).

Unter einem oxidierend wirkenden Gasgemisch G soll in dieser Schrift ein Gasgemisch G verstanden werden, das bei Anwendung des erfindungsgemäßen Verfahrens beim Eintritt in das Katalysatorfestbett die Oxidationszustände der in der multimetalloxidischen Aktivmasse enthaltenen Metalle nicht reduziert (sondern in der Regel oxidiert).

In der Regel ist ein Gasgemisch G daher dadurch gekennzeichnet, dass sein molarer Gehalt an molekularem Sauerstoff beim Eintritt des Gasgemischs G in das Katalysatorfestbett größer (vorzugsweise wenigstens doppelt so groß) ist als die Summe aus seinem molaren Gehalt an CO und seinen molaren Gehalten an von CO, Inertgas, O₂ und H₂O verschiedenen Bestandteilen.

Es überrascht, dass bei Anwendung des erfindungsgemäßen Verfahrens ein Langzeitbetrieb einer heterogen katalysierten Gasphasenpartialoxidation von Acrolein zu Acrylsäure möglich ist, mit dem die Ausprägung der Heißpunktausdehnung über die Zeit geringer ist als bei den Verfahren des Standes der Technik. In günstigen Fällen nimmt die Ausprägung der Heißpunktausdehnung über die Zeit sogar ab. Außerdem bleibt die Selektivität der Acrylsäurebildung über die Zeit meist weitgehend konstant.

Erfindungsgemäß bevorzugt wird man bereits bevor die Temperaturerhöhung des Katalysatorfestbetts dauerhaft ≥ 7°C, oder ≥ 6°C, oder ≥ 5°C, oder ≥ 4°C beträgt die Gasphasenpartialoxidation wenigstens einmal unterbrechen, um bei einer Temperatur des Katalysatorfestbetts von 200 bis 450°C ein an Acrolein freies, molekularen Sauerstoff, Inertgas und gegebenenfalls Wasserdampf sowie gegebenenfalls CO enthaltendes, oxidierend wirkendes Gasgemisch G durch das Katalysatorfestbett zu führen.

Besonders bevorzugt wird man beim erfindungsgemäßen Verfahren bereits bevor die Temperaturerhöhung des Katalysatorfestbetts dauerhaft ≥ 3°C, oder ≥ 2°C beträgt die Gasphasenpartialoxidation wenigstens einmal unterbrechen, um bei einer Temperatur des Katalysatorfestbetts von 200 bis 450°C ein an Acrolein freies, molekularen Sauerstoff, Inertgas und gegebenenfalls Wasserdampf sowie gegebenenfalls CO enthaltendes, oxidierend wirkendes Gasgemisch G durch das Katalysatorfestbett zu führen.

Das erfindungsgemäße Verfahren ist aber auch dann vorteilhaft, wenn, bevor die Temperaturerhöhung des Katalysatorfestbetts dauerhaft ≥ 1 °C oder weniger beträgt, die Gasphasenpartialoxidation wenigstens einmal unterbrochen und bei einer Temperatur von 200 bis 450°C ein an Acrolein freies, molekularen Sauerstoff, Inertgas und gegebenenfalls Wasserdampf sowie gegebenenfalls CO enthaltendes, oxidierend wirkendes Gasgemisch G durch das Katalysatorfestbett geführt wird. In der Regel wird die Temperaturerhöhung des Katalysatorfestbetts jedoch dauerhaft ≥ 0,1 °C oder ≥ 0,2°C betragen, bevor die Gasphasenpartialoxidation wenigstens einmal erfindungsgemäß unterbrochen wird.

Bevorzugt wird man die Temperatur des Katalysatorfestbetts während der Durchführung des Gasgemischs G bei der Ausführung des erfindungsgemäßen Verfahrens auf einem Wert T_{G} halten, der im wesentlichen derjenigen Temperatur T_{V} des Katalysatorfestbetts entspricht, bei der die Partialoxidation betrieben wurde, bevor man sie unterbricht, um das Gasgemisch G erfindungsgemäß durch das Katalysatorfestbett zu führen.

D.h., erfindungsgemäß vorteilhaft gilt T_{G} = T_{V} ± 50°C bzw. T_{G} = T_{V} ± 20°C und ganz besonders vorteilhaft ist T_{G} = T_{V}. Normalerweise wird T_{V} im Bereich von 200 bis 400°C, häufig im Bereich von 220 bis 350°C liegen. Die Zeitdauer t_{G} während der beim erfindungsgemäßen Verfahren das Gasgemisch G durch das Katalysatorfestbett zu führen ist, wird in der Regel 2 h bzw. 6 h bis 120 h, häufig 12 h bis 72 h und vielfach 20 bis 40 h betragen. Sie kann jedoch auch 10 Tage und mehr betragen. In der Regel wird ein kleiner Sauerstoffgehalt des Gasgemischs G eine längere Zeitdauer t_{G} bedingen. Erhöhte Sauerstoffgehalte im Gasgemisch G sind erfindungsgemäß vorteilhaft.

Zweckmäßig wird man t_{G} wenigstens so lange bemessen, bis sich der Sauerstoffgehalt des Gasgemischs G beim Eintritt in das und beim Austritt aus dem Katalysatorfestbett voneinander nicht mehr unterscheidet.

In anwendungstechnisch zweckmäßiger Weise wird das Gasgemisch G (alle in dieser Schrift angegebenen Gehalte des Gasgemischs G beziehen sich, soweit nichts anderes erwähnt wird, auf den Eintritt des Gasgemischs G in das Katalysatorfestbett) beim erfindungsgemäßen Verfahren wenigstens 1 oder 2 Vol.-%, vorzugweise wenigstens 3 Vol.% und besonders bevorzugt wenigstens 4 Vol.-% Sauerstoff enthalten. In der Regel wird der Sauerstoffgehalt des Gasgemischs G jedoch ≤ 21 Vol.-% betragen. D.h., ein mögliches Gasgemisch G ist Luft. Ein anderes mögliches Gasgemisch G ist Magerluft. Dabei handelt es sich um an Sauerstoff entreicherte Luft. Erfindungsgemäß vorteilhaft ist Magerluft, die aus 3 bis 10, vorzugsweise 4 bis 6 Vol.% Sauerstoff und als Restmenge aus molekularem Stickstoff besteht. Häufig ist es vorteilhaft, wenn das Gasgemisch G neben molekularem Sauerstoff und Inertgas zusätzlich Wasserdampf enthält. Anwendungstechnisch zweckmäßig enthält das Gasgemisch G wenigstens 0,1 Vol.-%, häufig wenigstens 0,5 Vol.-% und oft wenigstens 1 Vol.-% an Wasserdampf. Normalerweise liegt der Wasserdampfgehalt des Gasgemischs G bei ≤ 75 Vol.-%. Der Inertgasanteil des Gasgemischs G liegt in der Regel bei ≤ 95 Vol.%, meist bei ≤ 90 Vol.%. Erfindungsgemäß geeignete Gasgemische G können somit z.B. aus 3 bis 20 Vol.-% molekularem Sauerstoff, 1 bis 75 Vol.% Wasserdampf und als Restmenge aus Inertgas bestehen. Bevorzugte Inertgase sind N₂ und CO₂. Insbesondere kommen für das erfindungsgemäße Verfahren alle in der EP-A 614 872 empfohlenen Gasgemische G in Betracht. Ebenfalls können für das erfindungsgemäße Verfahren alle in der EP-A 614 872 empfohlenen Regenerierbedingungen angewendet werden.

Der CO-Gehalt eines erfindungsgemäß zu verwendenden Gasgemischs G wird in der Regel 5 Vol.% nicht überschreiten. Häufig wird der CO-Gehalt bei Werten von ≤ 3Vol.-%, oder ≤ 2 Vol.-%, oder ≤ 1 Vol.-% liegen oder ganz verschwindend sein.

Als vorteilhaft hat es sich erwiesen, wenn beim erfindungsgemäßen Verfahren der CO-Gehalt C, den das Gasgemisch G bei seinem Eintritt in das Katalysatorfestbett aufweist, innerhalb der Zeitdauer t_{G} von einem von Null verschiedenen Ausgangswert A zum Zeitpunkt t_{G} = 0 abfällt.

Erfindungsgemäß bevorzugt fällt der CO-Gehalt C innerhalb der Zeitdauer t_{G} von seinem Ausgangswert A auf einen Wert E ab, der weniger als 50 %, bevorzugt weniger als 25 %, besonders bevorzugt weniger als 10 % oder weniger als 5 % des Ausgangswertes A beträgt. Ganz besonders bevorzugt fällt der CO-Gehalt C auf einen Wert E = 0.

In entsprechender Weise kann auch der CO₂-Gehalt des Gasgemischs G innerhalb der Zeitdauer t_{G} variieren.

Umgekehrt ist es für das erfindungsgemäße Verfahren vorteilhaft, wenn der Sauerstoffgehalt des Gasgemischs G innerhalb der Zeitdauer t_{G} ausgehend von einem niederen Wert auf einen höheren Wert angehoben wird.

In typischer Weise hat es sich als vorteilhaft erwiesen, mit einem Sauerstoffgehalt des Gasgemischs G von etwa 1 bis 3 Vol.-% zu beginnen und ihn innerhalb der Zeitdauer t_{G} auf einen Wert von bis zu 10 Vol.-%, vorzugsweise von bis zu 6 Vol.% anzuheben. Der Wasserdampfgehalt das Gasgemischs G wird hingegen innerhalb der Zeitdauer t_{G} häufig fallend gewählt. Anfangswerte bewegen sich vielfach bei bis zu 10 Vol.-%, während der Endwert häufig bei ≤ 3 Vol.% liegt.

Erfindungsgemäß zweckmäßige Gasgemische G sind daher Gasgemische G*, die enthalten:
1 bis 8 (vorzugsweise 3 bis 6) Vol.-% Sauerstoff,
0 bis 3 Vol.-% CO,
0 bis 5 Vol.-% CO₂,
0 bis 25 Vol.-% H₂O, und
wenigstens 55 Vol.-% N₂ (vorzugsweise besteht G aus diesen Bestandteilen, wobei Stickstoff dann die Restmenge bis zu 100 Vol.% bildet).

Bevorzugt nimmt innerhalb von t_{G} der H₂O-, sowie der CO- und der CO₂-Gehalt des Gasgemischs G* wie beschieben ab, während der O₂-Gehalt wie beschrieben zunimmt. Als besonders vorteilhaft hat es sich erwiesen, wenn das Gasgemisch G beim Eintritt in das Katalysatorfestbett > 0 bis ≤ 20 Gew.ppm, häufig ≤ 15 bzw. ≤ 10 oder ≤ 5 bzw. ≤ 1 Gew.ppm an gasförmigen Mo-enthaltenden Verbindungen wie z.B. Molybdänoxidhydrat aufweist. Dieser Gehalt kann z.B. dadurch eingestellt werden, dass man bei erhöhter Temperatur (z.B. 250 - 500°C) ein Wasserdampf enthaltendes Gasgemisch G vorab der erfindungsgemäßen Verwendung durch ein Mo-Oxid enthaltendes Bett leitet.

Die Menge des beim erfindungsgemäßen Verfahren durch das Katalysatorfestbett geführten Gasgemischs G kann 5 oder 100 bis 5000 Nl/l•h, vorzugsweise 20 oder 200 bis 2000 Nl/l•h betragen (Bezugsbasis ist dabei das Volumen des gesamten Katalysatorfestbetts, d.h., einschließlich gegebenenfalls mitverwendeter Abschnitte die ausschließlich aus Inertmaterial bestehen).

In der Regel wird man beim erfindungsgemäßen Verfahren die Durchführung des Gasgemischs G durch das Katalysatorfestbett bei einer Temperatur des Katalysatorfestbetts von 200 bis 450°C in einer Häufigkeit H von wenigstens einmal pro Kalenderjahr, vorzugsweise von wenigstens einmal pro Kalenderdreivierteljahr bzw. pro Kalenderhalbjahr, besonders bevorzugt von wenigstens einmal pro Kalenderquartal und besonders bevorzugt von wenigstens einmal pro Kalendermonat durchführen. Im übrigen wird man das Verfahren der heterogen katalysierten Gasphasenpartialoxidation von Acrolein zu Acrylsäure weitgehend kontinuierlich durchführen.

In anderen Worten ausgedrückt wird man beim erfindungsgemäßen Verfahren die Durchführung des Gasgemischs G durch das Katalysatorfestbett bei einer Temperatur des Katalysatorfestbetts von 200 bis 450°C wenigstens einmal innerhalb von 7500 oder 7000, oder 6000, bevorzugt wenigstens einmal innerhalb von 5500 oder 5000 und ganz besonders bevorzugt wenigstens einmal innerhalb von 4000, oder 3000 oder 2000 oder 1500, oder 1000, oder 500 Betriebsstunden der Partialoxidation durchführen. Eine häufige Durchführung des erfindungsgemäßen Verfahrens wirkt sich vorteilhaft aus.

Als Aktivmasse für die Katalysatoren des Katalysatorfestbetts eignen sich bekanntermaßen Multimetalloxide, die die Elemente Mo und V enthalten.

Erfindungsgemäß geeignete, Mo und V enthaltende, Multimetalloxidaktivmassen können beispielsweise der US-A 3775474, der US-A 3954855, der US-A 3893951 und der US-A 4339355 oder der EP-A 614872 bzw. EP-A 1 041 062 oder der WO 03/055835 bzw. WO 03/057653 entnommen werden.

Insbesondere eignen sich auch die Multimetalloxidaktivmassen der DE-A 10 325 487 sowie der DE-A 10 325 488.

Ferner eignen sich in besonderer Weise die Multimetalloxidmassen der EP-A 427 508, der DE-A 2 909 671, der DE-C 31 51 805, der DE-AS 2 626 887, der DE-A 43 02 991, der EP-A 700 893, der EP-A 714 700 und der DE-A 19 73 6105 als Aktivmassen für für das erfindungsgemäße Verfahren geeignete Festbettkatalysatoren. Besonders bevorzugt sind in diesem Zusammenhang die beispielhaften Ausführungsformen der EP-A 714 700 sowie der DE-A 19 73 6105.

Eine Vielzahl dieser für besagte Festbettkatalysatoren geeigneten Multimetalloxidaktivmassen lässt sich unter der allgemeinen Formel 1,

Mo₁₂VₐX¹_{b}X²_{c}X³_{d}X⁴ₑX⁵_{f}X⁶_{g}Oₙ (I),

in der die Variablen folgende Bedeutung haben:
- X¹ =: W, Nb, Ta, Cr und/oder Ce,
- X² =: Cu, Ni, Co, Fe, Mn und/oder Zn,
- X³ =: Sb und/oder Bi,
- X⁴ =: eines oder mehrere Alkalimetalle,
- X⁵ =: eines oder mehrere Erdalkalimetalle,
- X⁶ =: Si, Al, Ti und/oder Zr,
- a =: 1 bis 6,
- b =: 0,2 bis 4,
- c =: 0,5 bis 18,
- d =: 0 bis 40,
- e =: 0 bis 2,
- f =: 0 bis 4,
- g =: 0 bis 40 und
- n =: eine Zahl, die durch die Wertigkeit und Häufigkeit der von Sauerstoff verschiedenen Elemente in I bestimmt wird,
subsummieren.

Bevorzugte Ausführungsformen innerhalb der aktiven Multimetalloxide I sind jene, die von nachfolgenden Bedeutungen der Variablen der allgemeinen Formel I erfasst werden:
- X¹ =: W, Nb, und/oder Cr,
- X² =: Cu, Ni, Co, und/oder Fe,
- X³ =: Sb,
- X⁴ =: Na und/oder K,
- X⁵ =: Ca, Sr und/oder Ba,
- X⁶ =: Si, Al, und/oder Ti,
- a =: 1,5 bis 5,
- b =: 0,5 bis 2,
- c =: 0,5 bis 3,
- d =: 0 bis 2,
- e =: 0 bis 0,2,
- f =: 0 bis 1 und
- n =: eine Zahl, die durch die Wertigkeit und Häufigkeit der von Sauerstoff verschiedenen Elemente in I bestimmt wird.

Ganz besonders bevorzugte Multimetalloxide I sind erfindungsgemäß jedoch jene der allgemeinen Formel II,

Mo₁₂V_{a'}Y¹_{b'}Y²_{c'}Y⁵_{f'}Y⁶_{g'}Oₙ (II),

mit
- Y¹ =: W und/oder Nb,
- Y² =: Cu und/oder Ni,
- Y⁵ =: Ca und/oder Sr,
- Y⁶ =: Si und/oder Al,
- a'=: 2 bis 4,
- b' =: 1 bis 1,5,
- c' =: 1 bis 3,
- f' =: 0 bis 0,5
- g' =: 0 bis 8 und
- n' =: eine Zahl, die durch die Wertigkeit und Häufigkeit der von Sauerstoff verschiedenen Elementen in II bestimmt wird.

Die erfindungsgemäß geeigneten Multimetalloxidaktivmassen (I) sind in sich bekannter, z.B. in der DE-A 4335973 oder in der EP-A 714700 offenbarter, Weise erhältlich. Insbesondere eignen sich aber auch die Multimetalloxidaktivmassen der DE-A 10 261 186.

Prinzipiell können für für das erfindungsgemäße Verfahren zu verwendende Festbettkatalysatoren geeignete Multimetalloxidaktivmassen, insbesondere solche der allgemeinen Formel I, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die für für das erfindungsgemäße Verfahren zu verwendende Festbettkatalysatoren geeigneten Multimetalloxidaktivmassen, insbesondere jene der allgemeinen Formel I, können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 3 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für für das erfindungsgemäße Verfahren geeignete Festbettkatalysatoren zu verwendende Multimetalloxidaktivmassen sind ferner Massen der allgemeinen Formel III,

[D]ₚ[E]_{q} (III),

in der die Variablen folgende Bedeutung haben:
- D =: Mo₁₂V_{a"}Z¹_{b"}Z²_{c"}Z³_{d"}Z⁴_{e"}Z⁵_{f"}Z⁶_{9"}O_{x"},
- E =: Z⁷₁₂C_{h"}H_{i"}O_{y"},
- Z¹ =: W, Nb, Ta, Cr und/oder Ce,
- Z² =: Cu, Ni, Co, Fe, Mn und/oder Zn,
- Z³ =: Sb und/oder Bi,
- Z⁴ =: Li, Na, K, Rb, Cs und/oder H
- Z⁵ =: Mg, Ca, Sr und/oder Ba,
- Z⁶ =: Si, Al, Ti und/oder Zr,
- Z⁷ =: Mo, W, V, Nb und/oder Ta,

- a" =: 1 bis 8,
- b" =: 0,2 bis 5,
- c" =: 0 bis 23,
- d" =: 0 bis 50,
- e" =: 0 bis 2,
- f" =: 0 bis 5,
- g" =: 0 bis 50,
- h" =: 4 bis 30,
- i" =: 0 bis 20 und
- x",y" =: Zahlen, die durch die Wertigkeit und Häufigkeit der von Sauerstoff verschiedenen Element in III bestimmt werden und
- p,q =: von Null verschiedene Zahlen, deren Verhältnis p/q 160:1 bis 1:1 beträgt,
und die dadurch erhältlich sind, dass man eine Multimetalloxidmasse E,

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D,

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind jene Multimetalloxidaktivmassen III, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen III-Katalysatoren enthalten z.B. die EP-A 668104, die DE-A 19736105 und die DE-A 19528646.

Hinsichtlich der Formgebung gilt bezüglich Multimetalloxidaktivmassen III-Katalysatoren das bei den Multimetalloxidaktivmassen I-Katalysatoren Gesagte.

Günstige Multimetalloxidaktivmassen für die Katalysatoren des Katalysatorfestbetts des erfindungsgemäßen Verfahrens sind ferner Multielementoxidaktivmassen der allgemeinen Formel IV,

[A]ₚ[B]_{q}[C]ᵣ (IV),

in der die Variablen folgende Bedeutung haben:
- A =: Mo₁₂VₐX¹_{b}X²_{c}X³_{d}X⁴ₑX⁵_{f}X⁶_{g}Oₓ,
- B =: X₁⁷CuₕHᵢO_{y},
- C =: X₁⁸SbⱼHₖO_{z},
- X¹ =: W, Nb, Ta, Cr und/oder Ce, vorzugsweise W, Nb und/oder Cr,
- X² =: Cu, Ni, Co, Fe, Mn und/oder Zn, vorzugsweise Cu, Ni, Co und/oder Fe,
- X³ =: Sb und/oder Bi, vorzugsweise Sb,
- X⁴ =: Li, Na, K, Rb, Cs und/oder H. vorzugsweise Na und/oder K,
- X⁵ =: Mg, Ca, Sr und/oder Ba, vorzugsweise Ca, Sr und/oder Ba,
- X⁶ =: Si, Al, Ti und/oder Zr, vorzugsweise Si, Al und/oder Ti,
- X⁷ =: Mo, W, V, Nb und/oder Ta, vorzugsweise Mo und/oder W,
- X⁸ =: Cu, Ni, Zn, Co, Fe, Cd, Mn, Mg, Ca, Sr und/oder Ba, vorzugsweise Cu und/oder Zn, besonders bevorzugt Cu,

- a =: 1 bis 8, vorzugsweise 2 bis 6,
- b =: 0,2 bis 5, vorzugsweise 0,5 bis 2,5
- c =: 0 bis 23, vorzugsweise 0 bis 4,
- d =: 0 bis 50, vorzugsweise 0 bis 3,
- e =: 0 bis 2, vorzugsweise 0 bis 0,3,
- f =: 0 bis 5, vorzugsweise 0 bis 2,
- g =: 0 bis 50, vorzugsweise 0 bis 20,
- h =: 0,3 bis 2,5, vorzugsweise 0,5 bis 2, besonders bevorzugt 0,75 bis 1,5,
- i =: 0 bis 2, vorzugsweise 0 bis 1,
- j =: 0,1 bis 50, vorzugsweise 0,2 bis 20, besonders bevorzugt 0,2 bis 5,
- k =: 0 bis 50, vorzugsweise 0 bis 20, besonders bevorzugt 0 bis 12,
- x,y,z =: Zahlen, die durch die Wertigkeit und Häufigkeit der von Sauerstoff verschiedenen Elementen in A, B, C bestimmt werden,
- p, q =: positive Zahlen
- r =: 0 oder eine positive Zahl, vorzugsweise eine positive Zahl, wobei das Verhältnis p/(q+r) = 20:1 bis 1:20, vorzugsweise 5:1 bis 1:14 und besonders bevorzugt 2:1 bis 1:8 und , für den Fall, dass r eine positive Zahl ist, das Verhältnis q/r = 20:1 bis 1:20, vorzugsweise 4:1 bis 1:4, besonders bevorzugt 2:1 bis 1:2 und ganz besonders bevorzugt 1:1 beträgt,
die den Anteil [A]p in Form dreidimensional ausgedehnter Bereiche (Phasen) A der chemischen Zusammensetzung
A: Mo₁₂VₐX¹_{b}X²_{c}X³_{d}X⁴ₑX⁵_{f}X⁶_{g}Oₓ,
den Anteil [B]q in Form dreidimensional ausgedehnter Bereiche (Phasen) B der chemischen Zusammensetzung
B : X₁⁷CuₕHᵢO_{y} und
den Anteil [C]ᵣ in Form dreidimensional ausgedehnter Bereiche (Phasen) C der chemischen Zusammensetzung
C: X₁⁸SbⱼHₖO_{z}
enthalten, wobei die Bereiche A, B und gegebenenfalls C relativ zueinander wie in einem Gemisch aus feinteiligem A, feinteiligem B und gegebenenfalls feinteiligem C verteilt sind, und wobei alle Variablen innerhalb der vorgegebenen Bereiche mit der Maßgabe auszuwählen sind, dass der molare Anteil des Elements Mo an der Gesamtmenge aller von Sauerstoff verschiedenen Elemente der Multielementoxidaktivmasse IV 20-mol% bis 80 mol-% beträgt, das molare Verhältnis von in der katalytisch aktiven Multielementoxidmasse IV enthaltenem Mo zu in der katalytisch aktiven Multielemetoxidmasse IV enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Bevorzugte Multielementoxidaktivmassen IV sind solche, deren Bereiche A eine Zusammensetzung im nachfolgenden Stöchiometrieraster der allgemeinen Formel V aufweisen,

Mo₁₂VₐX¹_{b}X²_{c}X⁵_{f}X⁶_{g}Oₓ (V),

mit
- X¹ =: W und/oder Nb,
- X² =: Cu und/oder Ni,
- X⁵ =: Ca und/oder Sr,
- X⁶ =: Si und/oder Al,
- a =: 2 bis 6,
- b =: 1 bis 2,
- c =: 1 bis 3,
- f =: 0 bis 0,75,
- g =: 0 bis 10, und
- x =: eine Zahl die durch die Wertigkeit und Häufigkeit der von Sauerstoff verschiedenen Elemente in (V) bestimmt wird.

Der im Zusammenhang mit den Multielementoxidaktivmassen V verwendete Begriff "Phase" meint dreidimensional ausgedehnte Bereiche, deren chemische Zusammensetzung von der ihrer Umgebung verschieden ist. Die Phasen sind nicht notwendigerweise röntgenographisch homogen. In der Regel bildet die Phase A eine kontinuierliche Phase, in der Partikel der Phase B und gegebenenfalls C dispergiert sind.

Die feinteiligen Phasen B und gegebenenfalls C bestehen mit Vorteil aus Partikeln, deren Größtdurchmesser, d.h., die längste durch den Schwerpunkt der Partikel gehende Verbindungsstrecke zweier auf der Oberfläche der Partikel befindlicher Punkte bis zu 300 µm, vorzugsweise 0,1 bis 200 µm, besonders bevorzugt 0,5 bis 50 µm und ganz besonders bevorzugt 1 bis 30 µm beträgt. Geeignet sind aber auch Partikel mit einem Größtdurchmesser von 10 bis 80 µm oder 75 bis 125 µm.

Prinzipiell können die Phasen A, B und gegebenenfalls C in den Multielementoxidaktivmassen IV amorph und/oder kristallin vorliegen.

Die den Multielementoxidaktivmassen der allgemeinen Formel IV zugrunde liegenden und anschließend zur Wandlung in Aktivmassen thermisch zu behandelnden innigen Trockengemische können z.B. so erhalten werden, wie es in den Schriften WO 02/24327, DE-A 4405514, DE-A 4440891, DE-A 19528646, DE-A 19740493, EP-A 756894, DE-A 19815280, DE-A 19815278, EP-A 774297, DE-A 19815281, EP-A 668104 und DE-A 19736105 beschrieben ist.

Das Grundprinzip der Herstellung von innigen Trockengemischen, die bei thermischer Behandlung zu Multielementoxidativmassen der allgemeinen Formel IV führen, besteht darin, wenigstens eine Multielementoxidmasse B (X₁⁷CuₕHᵢO_{y}) als Ausgangsmasse 1 und gegebenenfalls eine oder mehrere Multielementoxidmassen C (X₁⁸SbⱼHₖO_{z}) als Ausgangsmasse 2 entweder voneinander getrennt oder miteinander vergesellschaftet in feinteiliger Form vorzubilden und anschließend die Ausgangsmassen 1 und gegebenenfalls 2 mit einem Gemisch, das Quellen der elementaren Konstituenten der Multielementoxidmasse A

Mo₁₂VₐX_{b}¹X_{c}²X_{d}³Xₑ⁴X_{f}⁵X_{g}⁶Oₓ (A),

in einer der Stöchiometrie A entsprechenden Zusammensetzung enthält, im gewünschten Mengenverhältnis (gemäß der allgemeinen Formel IV) in innigen Kontakt zu bringen und die dabei resultierende innige Mischung gegebenenfalls zu trocknen.

Das innige in Kontakt bringen der Bestandteile der Ausgangsmassen 1 sowie gegebenenfalls 2 mit dem die Quellen der elementaren Konstituenten der Multimetalloxidmasse A enthaltenden Gemisch (Ausgangsmasse 3) kann sowohl trocken als auch nass erfolgen. Im letzteren Fall muss lediglich darauf geachtet werden, dass die vorgebildeten Phasen (Kristallite) B und gegebenenfalls C nicht in Lösung gehen. In wässrigem Medium ist letzteres bei pH-Werten, die nicht zu stark von 7 abweichen und bei nicht zu hohen Temperaturen üblicherweise gewährleistet. Erfolgt das innige in Kontakt bringen nass, wird abschließend normalerweise zum erfindungsgemäß thermisch zu behandelnden innigen Trockengemisch getrocknet (z.B. durch Sprühtrocknen). Im Rahmen eines trockenen Mischens fällt eine solche Trockenmasse automatisch an. Selbstredend können die feinteilig vorgebildeten Phasen B und gegebenenfalls C auch in ein plastisch verformbares Gemisch, das die Quellen der elementaren Konstituenten der Multimetalloxidmasse A enthält, eingearbeitet werden, wie es die DE-A 10046928 empfiehlt. Natürlich kann das innige in Kontakt bringen der Bestandteile der Ausgangsmassen 1 sowie gegebenenfalls 2 mit den Quellen der Multielementoxidmasse A (Ausgangsmasse 3) auch so erfolgen, wie es die DE-A 19815281 beschreibt.

Die thermische Behandlung zum Erhalt der Aktivmasse und die Formgebung kann wie bei den Multimetalloxidaktivmassen I bis III beschrieben erfolgen.

Ganz generell können Multimetalloxidaktivmassen I bis IV-Katalysatoren mit Vorteil gemäß der Lehre der DE-A 10 325 487 bzw. DE-A 10 325 488 hergestellt werden.

Anwendungstechnisch zweckmäßig wird man das erfindungsgemäße Verfahren der heterogen katalysierten Gasphasenpartialoxidation von Acrolein zu Acrylsäure in einem mit den Festbettkatalysatoren beschickten Rohrbündelreaktor, wie er z.B. in der EP-A 700 893 bzw. der DE-A 4 431 949 oder der WO 03/057653, oder der WO 03/055835, oder der WO 03/059857, oder der WO 03/076373 beschrieben ist, durchführen.

D.h., in einfachster Weise befindet sich das beim erfindungsgemäßen Verfahren zu verwendende Katalysatorfestbett in den einheitlich beschickten Metallrohren eines Rohrbündelreaktors und um die Metallrohre wird ein Temperiermedium (Einzonenfahrweise), in der Regel eine Salzschmelze, geführt. Salzschmelze (Temperiermedium) und Reaktionsgasgemisch können dabei im einfachen Gleich- oder Gegenstrom geführt werden. Das Temperiermedium (die Salzschmelze) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemischs besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor 0 bis 10°C, häufig 2 bis 8°C, oft 3 bis 6°C beträgt. Die Eintrittstemperatur des Wärmeaustauschmittels in den Rohrbündelreaktor (sie entspricht in dieser Schrift der Temperatur des Katalysatorfestbetts) beträgt in der Regel 220 bis 350°C, häufig 245 bis 285°C bzw. 245 bis 265°C. Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle. Aber auch ionische Flüssigkeiten sind einsetzbar.

Zweckmäßigerweise wird das Reaktionsgasgemisch der Beschickung mit Festbettkatalysator auf die gewünschte Reaktionstemperatur vorerwärmt zugeführt.

Insbesondere im Fall von angestrebten hohen (z.B. ≥ 140 Nl/l•h, in der Regel jedoch ≤ 600 Nl/l•h) Endbelastungen des Katalysatorfestbetts mit Acrolein erfolgt die Durchführung des erfindungsgemäßen Verfahrens zweckmäßig in einem Zweizonenrohrbündelreaktor (eine Durchführung im Einzonenrohrbündelreaktor ist jedoch ebenfalls möglich). Eine bevorzugte Variante eines für diesen Zweck erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind geeignet.

D.h., in einfacher Weise befindet sich das erfindungsgemäß zu verwendende Katalysatorfestbett in den einheitlich beschickten Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert eine Temperatur- bzw. Reaktionszone.

Vorzugsweise umströmt z.B. ein Salzbad A denjenigen Abschnitt der Rohre (die Reaktionszone A), in welchem sich die oxidative Umsetzung des Acroleins (beim einfachen Durchgang) bis zum Erreichen eines Umsatzwertes im Bereich von 55 bis 85 mol.% vollzieht und ein Salzbad B umströmt den Abschnitt der Rohre (die Rekationszone B), in welchem sich die oxidative Anschlußumsetzung des Acroleins (beim einfachen Durchgang) bis zum Erreichen eines Umsatzwertes von in der Regel wenigstens 90 mol.% vollzieht (bei Bedarf können sich an die Reaktionszonen A, B weitere Reaktionszonen anschließen, die auf individuellen Temperaturen gehalten werden).

Innerhalb der jeweiligen Temperaturzone kann das Salzbad prinzipiell wie bei der Einzonenfahrweise geführt werden. Die Eintrittstemperatur des Salzbades B liegt normalerweise wenigstens 5 bis 10°C oberhalb der Temperatur des Salzbades A. Im übrigen können die Eintrittstemperaturen im für die Einzonenfahrweise empfohlenen Temperaturbereich für die Eintrittstemperatur liegen.

Ansonsten kann die Zwei-Zonen-Hochlastfahrweise z.B. wie in der DE-A 19948523, der EP-A 1106598 oder wie in der DE-A 19948248 beschrieben durchgeführt werden.

Danach eignet sich das erfindungsgemäße Verfahren für Acroleinbelastungen des Katalysatorfestbetts von ≥ 70 Nl/l•h, ≥ 90 Nl/l•h, ≥ 110 Nl/l•h, ≥ 130 Nl/l•h, ≥ 180 Nl/l•h, ≥ 240 Nl/l•h, ≥ 300 Nl/l•h, jedoch normalerweise ≤ 600 Nl/l•h. Hier (d.h., generell bei Acroleinbelastungen in dieser Schrift), anders als sonst in dieser Schrift, ist die Belastung auf das Volumen des Katalysatorfestbetts ausschließlich gegebenenfalls mitverwendeter Abschnitte die ausschließlich aus Inertmaterial bestehen bezogen.

Dabei kann das für das Beschickungsgasgemisch zu verwendende Inertgas zu z.B. ≥ 20 Vol.-%, oder zu ≥ 30 Vol.-%, oder zu ≥ 40 Vol.-%, oder zu ≥ 50 Vol.%, oder zu ≥ 60 Vol.-%, oder zu ≥ 70 Vol.%, oder zu ≥ 80 Vol.-%, oder zu ≥ 90 Vol.%, oder zu ≥ 95 Vol.-% aus molekularem Stickstoff bestehen.

Ist die Gasphasenpartialoxidation des Acroleins die zweite Reaktionsstufe einer zweistufigen Gasphasenpartialoxidation von Propen zu Acrylsäure, wird das inerte Verdünnungsgas häufig zu 5 bis 25 bzw. 20 Gew.-% aus H₂O (wird in der ersten Reaktionsstufe gebildet und gegebenenfalls zugesetzt) und zu 70 bis 90 Vol.-% aus N₂ bestehen.

Bei Acroleinbelastungen des Katalysatorfestbetts oberhalb von 250 Nl/l•h wird für das erfindungsgemäße Verfahren jedoch die Mitverwendung von inerten Verdünnungsgasen wie Propan, Ethan, Methan, Butan, Pentan, CO₂, CO, Wasserdampf und/oder Edelgasen empfohlen. Selbstverständlich können diese Gase aber auch bereits bei geringeren Acroleinbelastungen mitverwendet werden.

Der Arbeitsdruck kann bei der erfindungsgemäßen Gasphasenpartialoxidation des Acroleins sowohl unterhalb von Normaldruck (z.B. bis zu 0,5 bar) als auch oberhalb von Normaldruck liegen. Typischerweise wird der Arbeitsdruck bei der Gasphasen-Partialoxidation des Acroleins bei Werten von 1 bis 5 bar, häufig 1 bis 3 bar liegen.

Normalerweise wird der Reaktionsdruck bei der erfindungsgemäßen Acroleinpartialoxidation 100 bar nicht überschreiten.

Das molare Verhältnis von O₂:Acrolein im Reaktionsgasausgangsgemisch, das beim erfindungsgemäßen Verfahren durch das Katalysatorfestbett geführt wird, wird normalerweise ≥ 1 betragen. Üblicherweise wird dieses Verhältnis bei Werten ≤ 3 liegen. Häufig wird das molare Verhältnis von O₂:Acrolein im vorgenannten Beschickungsgasgemisch erfindungsgemäß 1 bis 2 bzw. 1 bis 1,5 betragen. Vielfach wird man das erfindungsgemäße Verfahren mit einem im Reaktionsgasausgangsgemisch vorliegenden Acrolein:Sauerstoff:Wasserdampf:Inertgas-Volumenverhältnis (NI) von 1:(1 bis 3) : (0 bis 20) : (3 bis 30), vorzugsweise von 1 :(1 bis 3) : (0,5 bis 10) : (7 bis 10) ausführen.

Der Acroleinanteil im Reaktionsgasausgangsgemisch kann z.B. bei Werten von 3 oder 6 bis 15 Vol.-%, häufig bei 4 oder 6 bis 10 Vol.-% bzw. 5 bis 8 Vol.-% liegen (jeweils bezogen auf das Gesamtvolumen).

Zur Bereitung des Katalysatorfestbetts können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden (aus Inertmaterial bestehenden), Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

In der Regel ist es günstig, wenn sich die chemische Zusammensetzung der verwendeten Aktivmasse über das Katalysatorfestbett nicht verändert. D.h., die für einen einzelnen Katalysatorformkörper verwendete Aktivmasse kann zwar ein Gemisch aus verschiedenen die Elemente Mo und V enthaltenden Multimetalloxiden sein, für alle Katalysatorformkörper des Katalysatorfestbetts ist dann jedoch vorteilhaft das gleiche Gemisch zu verwenden.

Bevorzugt nimmt normalerweise die volumenspezifische (d.h., die auf die Einheit des Volumens normierte) Aktivität innerhalb des Katalysatorfestbetts in Strömungsrichtung des Reaktionsgasausgangsgemischs kontinuierlich, abrupt oder stufenförmig zu.

Die volumenspezifische Aktivität kann dabei z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen des Festbetts enthaltene Aktivmasse bzw. Katalysatoraktivität.

Eine in Strömungsrichtung des Reaktionsgasgemischs über des Katalysatorfestbett wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für das Katalysatorfestbett aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Vorab und/oder im Anschluss an die Aktivmasse aufweisenden Abschnitte des Katalysatorfestbetts können sich ausschließlich aus Inertmaterial (z.B. nur Verdünnungsformkörpern) bestehende Schüttungen befinden (sie werden in dieser Schrift begrifflich, soweit nichts anderes gesagt wird, dem Katalysatorfestbett zugerechnet). Diese können ebenfalls auf die Temperatur des Katalysatorfestbetts gebracht sein. Dabei können die für die Inertschüttung verwendeten Verdünnungsformkörper die gleiche Geometrie wie die zu den Aktivmasse aufweisenden Abschnitte des Katalysatorfestbetts verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Vielfach ist beim erfindungsgemäßen Verfahren der Aktivmasse aufweisende Abschnitt des Katalysatorfestbetts in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Aktivmasse aufweisenden Abschnitts der Festbettkatalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone befindet sich dann häufig vorteilhaft bis zum Ende der Länge des Aktivmasse aufweisenden Abschnitts des Katalysatorfestbetts (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn im Katalysatorfestbett als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende des Katalysatorfestbetts.

Eine reine Inertmaterialschüttung, deren Länge, bezogen auf die Gesamtlänge des Katalysatorfestbetts, zweckmäßig 5 bis 20 % beträgt, leitet in Strömungsrichtung des Reaktionsgasgemischs in der Regel das Katalysatorfestbett ein. Sie wird normalerweise als Aufheizzone für das Reaktionsgasgemisch genutzt.

Üblicherweise sind die Kontaktrohre in den Rohrbündelreaktoren aus ferritischem Stahl gefertigt und weisen in typischer Weise eine Wanddicke von 1 bis 3 mm auf. Ihr Innendurchmesser beträgt in der Regel (einheitlich) 20 bis 30 mm, häufig 21 bis 26 mm. Anwendungstechnisch zweckmäßig beläuft sich die im Rohrbündelbehälter untergebrachte Anzahl an Kontaktrohen auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Die Belastung des Katalysatorfestbetts (hier ausschließlich reiner Inertabschnitte) mit Reaktionsgasgemisch wird beim erfindungsgemäßen Verfahren in typischer Weise 1000 bis 10000 Nl/l•h, meist 1000 bis 5000 Nl/l•h, häufig 1500 bis 4000 Nl/l•h betragen.

Bei der Ausübung des erfindungsgemäßen Verfahrens wird man ein frisches Katalysatorfestbett nach seiner Formierung normalerweise so betreiben, dass man nach Festlegung der Zusammensetzung des Reaktionsgasgemischs und Festlegung der Belastung des Katalysatorfestbetts mit Reaktionsgasgemisch die Temperatur des Katalysatorfestbetts (bzw. die Eintrittstemperatur des Temperiermediums in die Temperierzone des Rohrbündelreaktors) so einstellt, dass der Umsatz U^{Acr} des Acroleins bei einmaligem Durchgang des Reaktionsgasgemischs durch das Katalysatorfestbett wenigstens 90 mol.-% beträgt. Bei Verwendung günstiger Katalysatoren sind auch Werte für U^{Acr} ≥ 92 mol.-%, oder ≥ 94 mol.-%, oder ≥ 96 mol.-%, oder ≥ 98 mol.-%, und häufig sogar ≥ 99 mol.% und mehr möglich.

Bei kontinuierlicher Ausübung der heterogen katalysierten Partialoxidation von Acrolein zu Acrylsäure wird man die Zusammensetzung des Reaktionsgasausgangsgemischs und die Belastung des Katalysatorfestbetts mit Reaktionsgasausgangsgemisch im wesentlichen konstant beibehalten (gegebenenfalls wird die Belastung an die fluktuierende Marktnachfrage angepasst). Einem Abfallen der Aktivität des Katalysatorfestbetts über die Zeit wird man unter diesen Produktionsbedingungen normalerweise dadurch entgegenwirken, dass man von Zeit zu Zeit die Temperatur des Katalysatorfestbetts (die Eintrittstemperatur des Temperiermediums in die Temperaturzone des Rohrbündelreaktors) erhöht (die Fließgeschwindigkeit des Temperiermediums wird normalerweise im wesentlichen ebenfalls beibehalten), um den Acroleinumsatz bei einmaligem Durchgang des Reaktionsgasgemischs im angestrebten Zielkorridor (d.h., bei Werten von ≥ 90 mol-%, bzw. ≥ 92 mol-%, bzw. ≥ 94 mol-%, bzw. ≥ 96 mol-%, bzw. ≥ 98 mol-%, bzw. ≥ 99 mol-%) zu halten. Eine solche Vorgehensweise zieht jedoch die Eingangs dieser Schrift beschriebenen Nachteile mit sich.

Erfindungsgemäß vorteilhaft wird man deshalb so verfahren, dass man, bevor die vorgenommene Temperaturerhöhung des Katalysatorfestbetts dauerhaft ≥ 10°C oder ≥ 8°C beträgt (bezogen auf die zuvor eingestellte Temperatur des Katalysatorfestbetts), die Gasphasenpartialoxidation wenigstens einmal unterbricht, um bei einer Temperatur des Katalysatorfestbetts von 200 bis 450°C ein an Acrolein freies, molekularen Sauerstoff, Inertgas und gegebenenfalls Wasserdampf sowie gegebenenfalls CO enthaltendes, oxidierend wirkendes (z.B. aus molekularem Sauerstoff, Inertgas und gegebenenfalls Wasserdampf bestehendes) Gasgemisch G durch das Katalysatorfestbett zu führen. Anschließend wird die Partialoxidation unter weitgehendem Beibehalt der Verfahrensbedingungen (vorzugsweise wird die Acroleinbelastung des Katalysatorfestbetts wie bei einem frischen Katalysatorfestbett langsam wiedereingestellt, z.B. so wie in der DE-A 10337788 beschrieben) fortgesetzt und die Temperatur des Katalysatorfestbetts so eingestellt, dass der Acroleinumsatz den angestrebten Zielwert erreicht. In der Regel wird dieser Temperaturwert bei gleichem Umsatz bei einem etwas niedrigeren Wert liegen als die Temperatur, die das Katalysatorfestbett vor der Unterbrechung der Partialoxidation und der erfindungsgemäßen Behandlung mit dem Gasgemisch G aufwies. Von diesem Temperaturwert des Katalysatorfestbetts ausgehend wird die Partialoxidation unter weitgehendem Beibehalt der übrigen Bedingungen fortgesetzt und dabei dem Abfall der Aktivität des Katalysatorfestbetts über die Zeit in zweckmäßiger Weise wiederum dadurch entgegengewirkt, dass man von Zeit zu Zeit die Temperatur des Katalysatorfestbetts erhöht. Bevor die durchgeführte Temperaturerhöhung des Katalysatorfestbetts dauerhaft ≥ 10°C oder ≥ 8°C beträgt, wird die Partialoxidation erfindungsgemäß wiederum wenigstens einmal unterbrochen, um das Gasgemisch G in erfindungsgemäßer Weise durch das Katalysatorfestbett zu führen. Danach wird die Partialoxidation erfindungsgemäß vorteilhaft wie beschrieben wieder aufgenommen u.s.w..

Der Wortlaut "bevor die durchgeführte Temperaturerhöhung des Katalysatorfestbetts dauerhaft ≥ 10°C oder ≥ 8°C (allgemein ≥ X°C) beträgt" berücksichtigt dabei, dass die Temperatur des Katalysatorfestbetts im großtechnischen Betrieb aus unterschiedlichen Gründen gewissen Schwankungen unterliegen kann. In diesem Fall trägt man den tatsächlichen Verlauf der Temperatur des Katalysatorfestbetts über die Zeit auf und legt durch die Messpunkte nach der von Legendre und Gauß entwickelten Methode der kleinsten Summe der Abweichungsquadrate eine Ausgleichskurve. Ist auf dieser Ausgleichskurve eine Temperaturerhöhung von ≥ 10°C oder ≥ 8°C (allgemein von ≥ X°C) erreicht, gilt das Merkmal "dauerhaft" als erfüllt.

Es überrascht, dass beim erfindungsgemäßen Verfahren die Ausprägung der Heißpunktausdehnung im Langzeitbetrieb einer heterogen katalysierten Gasphasenpartialoxidation von Acrolein zu Acrylsäure ein günstigeres Verhalten zeigt, als bei den Verfahren gemäß des Standes der Technik. Das erfindungsgemäße Verfahren ermöglicht so einerseits längere Standzeiten eines Katalysatorfestbetts in einem Reaktor, bevor dieses teilweise oder vollständig ausgetauscht werden muss. Auf der anderen Seite ist auch der über die Zeit integral erzielte Acroleinumsatz ein erhöhter und die Selektivität der Acrylsäurebildung wird ebenfalls gefördert, da der Ort des Heißpunktes beim erfindungsgemäßen Verfahren über die Zeit normalerweise in Richtung der Eintrittsstelle des Reaktionsgasgemischs in das Katalysatorfestbett wandert. Damit wandert der Heißpunkt im Reaktionsgasgemisch zunehmend in den Bereich vor, in dem der Acrylsäuregehalt noch wenig ausgeprägt ist. Dies mindert die Möglichkeit, dass bereits gebildete Acrylsäure unter der Einwirkung der Heißpunkttemperatur partiell unerwünschte Vollverbrennung erleidet. Die Ermittlung der Heißpunkttemperatur kann beim erfindungsgemäßen Verfahren in Rohrbündelreaktoren z.B. mittels Thermorohren erfolgen, wie sie in der EP-A 873 783, der WO 03-076373 und in der EP-A 1 270 065 beschrieben sind. Die Anzahl solcher Thermorohre innerhalb eines Rohrbündelreaktors beträgt zweckmäßig 4 bis 20. Mit Vorteil befinden sie sich im Rohrbündelinneren gleichmäßig verteilt angeordnet.

Häufig wird man beim erfindungsgemäßen Verfahren die Erhöhung der Katalysatorfestbetttemperatur so durchführen, dass der Acroleinumsatz bei einmaligem Durchgang des Reaktionsgasgemischs durch das Katalysatorfestbett 90 mol-%, bzw. 92 mol-%, bzw. 94 mol-%, bzw. 96 mol-%, bzw. 98 mol-%, bzw. 99 mol-% nicht unterschreitet. D.h., normalerweise wird man die Katalysatorfestbetttemperatur wenigstens einmal erhöhen bevor 7500 oder 7000, meist bevor 6000 und vielfach bevor 5000 oder 4000 Betriebsstunden der Partialoxidation erreicht sind.

Abschließend sei festgehalten, dass die Erhöhung der Katalysatorfestbetttemperatur über die Zeit beim erfindungsgemäßen Verfahren unter Anwendung besonders günstiger Katalysatoren (z.B. den in dieser Schrift empfohlenen) bevorzugt (meist im wesentlichen kontinuierlich und) so durchgeführt wird, dass der Acroleingehalt im Produktgasgemisch den Wert 1500 Gew.-ppm, vorzugsweise 600 Gew.-ppm und besonders bevorzugt 350 Gew.-ppm nicht übersteigt. Dabei wird berücksichtigt, dass Acrolein bei den Verfahren der Abtrennung von Acrylsäure aus dem Produktgasgemisch der Partialoxidation insofern störend wirkt, als es die Polymerisationsneigung von Acrylsäure fördert (vgl. EP-A 1 041 062). Ferner sollte der Restsauerstoff im Produktgasgemisch in der Regel wenigstens 1 Vol.-%, bevorzugt wenigstens 2 Vol.-% und besonders bevorzugt wenigstens 3 Vol.-% betragen.

Das erfindungsgemäße Verfahren ist insbesondere dann vorteilhaft, wenn es mit einer Belastung des Katalysatorfestbetts mit Acrolein von ≥ 110 Nl/l•h, bzw. ≥ 120 Nl/l•h, bzw. ≥ 130 Nl/l•h betrieben wird. Generell wird man das frisch beschickte Katalysatorfestbett so gestalten, dass, wie in der EP-A 990636 und in der EP-A 1106598 beschrieben, sowohl die Heißpunktausbildung als auch deren Temperatursensitivität möglichst gering sind. Ferner wir man sowohl bei der Erstinbetriebnahme als auch bei der Wiedereinbetriebnahme nach Ausübung des erfindungsgemäßen Verfahrens die Belastung des Katalysatorfestbetts mit Acrolein vorteilhaft zunächst bei Werten ≤ 100 Nl/l·h belassen, bis sich ein stabiler Betrieb eingestellt hat.

### Beispiele

### A) Herstellung des verwendeten Katalysators

### 1. Allgemeine Beschreibung des zur Calcination im Rahmen der Herstellung von Ausgangsmassen 1 und 2 verwendeten Drehrohrofens

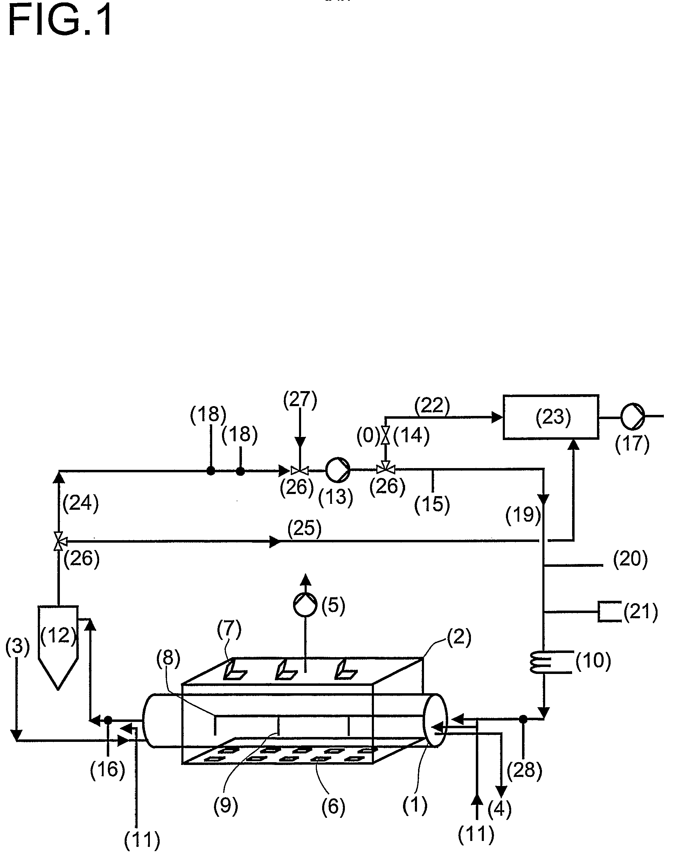

Eine schematische Darstellung des Drehrohrofens zeigt die dieser Schrift beiliegende Figur 1. Die nachfolgenden Bezugsziffern beziehen sich auf diese Figur 1.

Zentrales Element des Drehrohrofens ist das Drehrohr (1). Es ist 4000 mm lang und weist einen Innendurchmesser von 700 mm auf. Es ist aus Edelstahl 1.4893 gefertigt und weist eine Wanddicke von 10 mm auf.

Auf der Innenwand des Drehrohrofens sind Hublanzen angebracht, die eine Höhe von 5 cm und eine Länge von 23,5 cm aufweisen. Sie dienen primär dem Zweck, das thermisch im Drehrohrofen zu behandelnde Gut anzuheben und dadurch zu durchmischen.

Auf ein und derselben Höhe des Drehrohrofens sind um den Umfang äquidistant (jeweils 90° Abstand) jeweils vier Hublanzen angebracht (ein Quadrupel). Längs des Drehrohrofens befinden sich acht solcher Quadrupel (jeweils im Abstand von 23,5 cm). Die Hublanzen zweier benachbarter Quadrupel sitzen am Umfang gegeneinander versetzt. Am Anfang und am Ende Drehrohrofens (erste und letzte 23,5 cm) befinden sich keine Hublanzen.

Das Drehrohr rotiert frei in einem Quader (2), der vier in der Länge des Drehrohres aufeinanderfolgende, gleich lange, elektrisch beheizte (Widerstandsheizung) Heizzonen aufweist, von denen jede den Umfang des Drehrohrofens umschließt. Jede der Heizzonen kann den entsprechenden Drehrohrabschnitt auf Temperaturen zwischen Raumtemperatur und 850°C erhitzen. Die maximale Heizleistung jeder Heizzone beträgt 30 kW. Der Abstand zwischen elektrischer Heizzone und Drehrohraußenfläche beträgt etwa 10 cm. Am Anfang und am Ende ragt das Drehrohr ca. 30 cm aus dem Quader heraus.

Die Umdrehungsgeschwindigkeit kann zwischen 0 und 3 Umdrehungen pro Minute variabel eingestellt werden. Das Drehrohr ist sowohl links- als auch rechts-drehbar. Bei Rechtsdrehung verweilt das Materialgut im Drehrohr, bei Linksdrehung wird das Materialgut vom Eintrag (3) zum Austrag (4) gefördert. Der Neigungswinkel des Drehrohres zur Horizontalen kann zwischen 0° und 2° variabel eingestellt werden. Im diskontinuierlichen Betrieb liegt er faktisch bei 0°. Im kontinuierlichen Betrieb befindet sich die tiefstliegendste Stelle des Drehrohrs beim Materialgutaustrag. Eine Schnellkühlung des Drehrohres kann dadurch erfolgen, dass die elektrischen Heizzonen abgeschaltet und ein Ventilator (5) eingeschaltet wird. Dieser saugt durch im unteren Boden des Quaders befindliche Löcher (6) Umgebungsluft an, und fördert sie durch drei im Deckel befindliche Klappen (7) mit variabel einstellbarer Öffnung.

Der Materialguteintrag wird über eine Zellenradschleuse kontrolliert (Massenkontrolle). Der Materialgutaustrag wird, wie bereits erwähnt, über die Drehrichtung des Drehrohrs gesteuert.

Bei diskontinuierlichem Betrieb des Drehrohres kann eine Materialgutmenge von 250 bis 500 kg thermisch behandelt werden. Sie befindet sich dabei normalerweise ausschließlich im beheizten Teil des Drehrohres.

Von einer auf der zentralen Achse des Drehrohres liegenden Lanze (8) führen in Abständen von 800 mm insgesamt drei Thermoelemente (9) vertikal ins Materialgut. Sie ermöglichen die Bestimmung der Temperatur des Materialguts. In dieser Schrift wird unter Temperatur des Materialgutes das arithmetische Mittel der drei Thermoelementtemperaturen verstanden. Innerhalb des im Drehrohr befindlichen Materialguts beträgt die maximale Abweichung zweier gemessener Temperaturen erfindungsgemäß zweckmäßig weniger als 30°C, bevorzugt weniger als 20°C, besonders bevorzugt weniger als 10°C und ganz besonders bevorzugt weniger als 5 bzw. 3°C.

Durch das Drehrohr können Gasströme geführt werden, mittels derer sich die Calcinationsatmosphäre bzw. allgemein die Atmosphäre der thermischen Behandlung des Materialgutes einstellen lässt.

Der Erhitzer (10) bietet die Möglichkeit, den ins Drehrohr geführten Gasstrom vorab seines Eintritts ins Drehrohr auf die gewünschte Temperatur zu erwärmen (z.B. auf die für das Materialgut im Drehrohr gewünschte Temperatur). Die maximale Leistung des Erhitzers liegt bei 1 x 50 kW + 1 x 30 kW. Vom Prinzip her kann es sich beim Erhitzer (10) z.B. um einen indirekten Wärmetauscher handeln. Solch ein Erhitzer kann prinzipiell auch als Kühler genutzt werden. In der Regel handelt es sich aber um einen Elektroerhitzer, bei dem der Gasstrom über Strom beheizte Metalldrähte geführt wird (zweckmäßigerweise ein CSN Durchlauferhitzer, Typ 97D/80 der Fa. C. Schniewindt KG, 58805 Neuerade - DE).

Prinzipiell sieht die Drehrohrvorrichtung die Möglichkeit vor, den durch das Drehrohr geführten Gasstrom teilweise oder vollständig im Kreis zu führen. Die dazu erforderliche Kreisleitung wird am Drehrohreintritt und am Drehrohraustritt über Kugellager oder über Graphitpressdichtungen mit dem Drehrohr beweglich verbunden. Diese Verbindungen werden mit Inertgas (z.B. Stickstoff) gespült (Sperrgas). Die beiden Spülströme (11) ergänzen den durch das Drehrohr geführten Gasstrom am Eintritt ins Drehrohr und am Austritt aus dem Drehrohr. Zweckmäßigerweise verjüngt sich dabei das Drehrohr an seinem Anfang und an seinem Ende und ragt in das zu- bzw. wegführende Rohr der Kreisleitung hinein.

Hinter dem Austritt des durch das Drehrohr geführten Gasstroms befindet sich ein Zyklon (12), zur Abtrennung von mit dem Gasstrom mitgerissenen Feststoffpartikeln (der Zentrifugalabscheider trennt in der Gasphase suspendierte Feststoffpartikel durch Zusammenwirken von Zentrifugal- und Schwerkraft ab; die Zentrifugalkraft des als Spiralwirbel rotierenden Gasstroms beschleunigt die Sedimentation der suspendierten Teilchen).

Die Förderung des Kreisgasstroms (24) (die Gaszirkulation) erfolgt mittels eines Kreisgasverdichters (13) (Ventilators), der in Richtung des Zyklons ansaugt und in die andere Richtung drückt. Unmittelbar hinter dem Kreisgasverdichter liegt der Gasdruck in der Regel oberhalb einer Atmosphäre. Hinter dem Kreisgasverdichter befindet sich ein Kreisgasauslass (über ein Regelventil (14) kann Kreisgas ausgeschleust werden). Eine hinter dem Auslass befindliche Blende (Querschnittsverjüngung um etwa einen Faktor 3, Druckminderer) (15) erleichtert den Auslass.

Über das Regelventil lässt sich der Druck hinter dem Drehrohrausgang regulieren. Dies geschieht im Zusammenspiel mit einem hinter dem Drehrohrausgang angebrachten Drucksensor (16), dem Abgasverdichter (17) (Ventilator), der zum Regelventil hin gerichtet ansaugt, dem Kreisgasverdichter (13) und der Frischgaszufuhr. Relativ zum Außendruck kann der Druck (unmittelbar) hinter dem Drehrohrausgang z.B. bis zu +1,0 mbar oberhalb und z.B. bis zu -1,2 mbar unterhalb liegend eingestellt werden. D.h., der Druck des das Drehrohr durchströmenden Gasstroms kann beim Verlassen des Drehrohrs unterhalb des Umgebungsdrucks des Drehrohrs liegen.

Ist beabsichtigt, den durch das Drehrohr geführten Gasstrom auch nicht wenigstens anteilig im Kreis zu führen, wird die Verbindung zwischen Zyklon (12) und Kreisgasverdichter (13) nach dem Dreiwegehahnprinzip (26) geschlossen und der Gasstrom auf direktem Weg in die Abgasreinigungsvorrichtung (23) geführt. Die hinter dem Kreisgasverdichter befindliche Verbindung zur Abgasreinigungsvorrichtung wird in diesem Fall ebenfalls nach dem Dreiwegehahnprinzip geschlossen. Besteht der Gasstrom im wesentlichen aus Luft, wird diese in diesem Fall über den Kreisgasverdichter (13) angesaugt (27). Die Verbindung zum Zyklon ist nach dem Dreiwegehahnprinzip geschlossen. Vorzugsweise wird der Gasstrom in diesem Fall durch das Drehrohr gesaugt, so dass der Drehrohrinnendruck kleiner als der Umgebungsdruck ist.

Bei kontinuierlichem Betrieb der Drehrohrofenvorrichtung wird der Druck hinter dem Drehrohrausgang vorteilhaft -0,2 mbar unterhalb des Außendruckes liegend eingestellt. Bei diskontinuierlichem Betrieb der Drehrohrvorrichtung wird der Druck hinter dem Drehrohrausgang vorteilhaft -0,8 mbar unterhalb des Außendruckes liegend eingestellt. Der leichte Unterdruck dient dem Zweck einer Vermeidung einer Kontamination der Umgebungsluft mit Gasgemisch aus dem Drehrohrofen.

Zwischen dem Kreisgasverdichter und dem Zyklon befinden sich Sensoren (18), die zum Beispiel den Ammoniakgehalt und den Sauerstoffgehalt im Kreisgas bestimmen. Der Ammoniaksensor arbeitet bevorzugt nach einem optischen Messprinzip (die Absorption von Licht bestimmter Wellenlänge korreliert proportional zum Ammoniakgehalt des Gases) und ist zweckmäßig ein Gerät der Fa. Perkin & Elmer vom Typ MCS 100. Der Sauerstoffsensor fußt auf den paramagnetischen Eigenschaften des Sauerstoffs und ist zweckmäßigerweise ein Oximat der Fa. Siemens vom Typ Oxymat MAT SF 7MB1010-2CA01-1 AA1-Z.

Zwischen der Blende (15) und dem Erhitzer (10) können Gase wie Luft, Stickstoff, Ammoniak oder andere Gase zum tatsächlich rezirkulierten Kreisgasanteil (19) zudosiert werden. Häufig wird eine Grundlast an Stickstoff zudosiert (20). Mit einem separaten Stickstoff/Luft Splitter (21) kann auf den Messwert des Sauerstoffsensors reagiert werden.

Der ausgeschleuste Kreisgasanteil (22) (Abgas) enthält häufig nicht völlig unbedenkliche Gase wie NOₓ, Essigsäure, NH₃, u.s.w.), weshalb diese im Normalfall in einer Abgasreinigungsvorrichtung abgetrennt werden (23).

Dazu wird das Abgas in der Regel zunächst über eine Waschkolonne geführt (ist im wesentlichen eine an Einbauten freie Kolonne, die vor ihrem Ausgang eine trennwirksame Packung enthält; das Abgas und wässriger Sprühnebel werden im Gleichstrom und im Gegenstrom geführt (2 Sprühdüsen mit entgegengesetzter Sprührichtung).

Aus der Waschkolonne kommend wird das Abgas in eine Vorrichtung geführt, die einen Feinstaubfilter (in der Regel ein Bündel aus Schlauchfiltern) enthält aus dessen Innerem das eingedrungene Abgas weggeführt wird. Dann wird abschließend in einer Muffel verbrannt.

Mittels eines Sensors (28) der Fa. KURZ Instruments, Inc., Montery (USA) vom Typ Modell 455 Jr wird die Menge des vom Sperrgas verschiedenen, dem Drehrohr zugeführten, Gasstroms gemessen und geregelt (Messprinzip: thermalkonvektive Massendurchflussmessung unter Anwendung eines Gleichtemperatur-Anemometers).

Bei kontinuierlichem Betrieb werden Materialgut und Gasphase im Gegenstrom durch den Drehrohrofen geführt.

Stickstoff bedeutet im Zusammenhang mit diesem Beispiel immer Stickstoff mit einer Reinheit > 99 Vol.-%.

### 2. Herstellung der Ausgangsmasse 1 (Phase B) mit der Stöchiometrie Cu₁Mo_{0,5}W_{0,5}O₄

Zu 603 I Wasser wurden 98 I einer 25 gew.-%igen wässrigen NH₃-Lösung gegeben. Anschließend wurden in der wässrigen Mischung 100 kg Kupfer(II)acetathydrat (Gehalt: 40,0 Gew.-% CuO) gelöst, wobei eine klare, tiefblaue wässrige Lösung 1 entstand, die 3,9 Gew.-% Cu enthielt und Raumtemperatur aufwies.

Unabhängig von der Lösung 1 wurden 620 I Wasser auf 40°C erwärmt. Darin wurden unter Aufrechterhaltung der 40°C 27,4 kg Ammoniumheptamolybdattetrahydrat (81,5 Gew.-% MoO₃) innerhalb von 20 min. unter Rühren gelöst. Dann wurden 40,4 kg Ammoniumparawolframatheptahydrat (88,9 Gew.-% WO₃) zugefahren und nach Aufheizen auf 90°C innerhalb von 45 min. bei dieser Temperatur unter Rühren gelöst. Es wurde eine klare, gelb-orange gefärbte wässrige Lösung 2 erhalten.

Anschließend wurde die wässrige Lösung 1 in die 90°C aufweisende Lösung 2 eingerührt, wobei die Temperatur der Gesamtmischung nicht unter 80°C sank. Die resultierende wässrige Suspension wurde 30 min. bei 80°C nachgerührt. Danach wurde sie mit einem Sprühtrockner der Fa. Niro-Atomizer (Kopenhagen) vom Typ S-50-N/R sprühgetrocknet (Gaseintrittstemperatur: 315°C, Gasaustrittstemperatur: 110°C, Gleichstrom). Das Sprühpulver wies einen Partikeldurchmesser von 2 bis 50 µm auf.

100 kg von so erhaltenem hellgrünem Sprühpulver wurden in einen Kneter der Fa. AMK (Aachener Misch- und Knetmaschinen Fabrik) vom Typ VIU 160 (Sigma Schaufeln) dosiert und unter Zugabe von 8 I Wasser geknetet (Verweilzeit: 30 min, Temperatur: 40 bis 50°C). Danach wurde das Knetgut in einen Extruder entleert und mittels des Extruders (Fa. Bonnot Company (Ohio), Typ: G 103-10/D7A-572K (6" Extruder W/Packer) zu Strängen (Länge: 1-10 cm; Durchmesser: 6 mm) geformt. Auf einem Bandtrockner wurden die Stränge 1 h bei einer Temperatur von 120°C (Materialguttemperatur) getrocknet. Die getrockneten Stränglinge wurden anschließend im unter "1." beschriebenen Drehrohrofen wie folgt thermisch behandelt (calciniert):
- die thermisch Behandlung erfolgte kontinuierlich mit einem Materialguteintrag von 50 kg/h an Stränglingen;
- der Neigungswinkel des Drehrohres zur Horizontalen betrug 2°;
- im Gegenstrom zum Materialgut wurde ein Luftstrom von 75 Nm³/h durch das Drehrohr geführt, der von insgesamt (2 x 25) 50 Nm³/h Sperrgas der Temperatur 25°C ergänzt wurde;
- der Druck hinter dem Drehrohrausgang lag 0,8 mbar unter dem Außendruck;
- das Drehrohr rotierte mit 1,5 Umdrehungen/min links herum;
- es wurde keine Kreisgasfahrweise angewendet;
- beim ersten Durchgang der Stränglinge durch das Drehrohr wurde die Temperatur der Drehrohraußenwand auf 340°C eingestellt, der Luftstrom wurde mit einer Temperatur von 20 bis 30°C in das Drehrohr geführt;
- anschließend wurden die Stränglinge mit der selben Durchsatzmenge und abgesehen von den nachfolgenden Unterschieden unter den gleichen Bedingungen durch das Drehrohr geführt:
   - die Temperatur der Drehrohrwand wurde auf 790°C eingestellt;
   - der Luftstrom wurde auf eine Temperatur von 400°C erwärmt in das Drehrohr geführt.

Anschließend wurden die eine rotbraune Farbe aufweisenden Stränglinge auf einer Biplexquerstromsichtmühle (BQ 500) der Fa. Hosokawa-Alpine (Augsburg) auf einen mittleren Partikeldurchmesser von 3 bis 5 µm gemahlen. Die so erhaltene Ausgangsmasse 1 wies eine BET-Oberfläche ≤ 1 m²/g auf. Mittels Röntgenbeugung wurden folgende Phasen festgestellt:
1. CuMoO₄-III mit Wolframitstruktur;
2. HT-Kupfermolybdat.

### 3. Herstellung der Ausgangsmasse 2 (Phase C) mit der Stöchiometrie CuSb₂O₆

Unter Rühren wurden 52 kg Antimontrioxid (99,9 Gew.-% Sb₂O₃) in 216 I Wasser (25°C) suspendiert. Die resultierende wässrige Suspension wurde auf 80°C erwärmt. Anschließend wurde unter Aufrechterhaltung der 80°C 20 min nachgerührt. Anschließend wurden innerhalb einer Stunde 40 kg einer 30 gew.-%igen wässrigen Wasserstoffperoxidlösung zugegeben, wobei die 80°C aufrechterhalten wurden. Unter Beibehalt dieser Temperatur wurde 1,5 Stunden nachgerührt. Dann wurden 20 I 60°C warmes Wasser zugegeben und so eine wässrige Suspension 1 erhalten. In diese wurden bei einer Temperatur von 70°C 618,3 kg einer wässrigen ammoniakalischen Kupfer(II)acetatlösung (60,8 g Kupferacetat pro kg Lösung und 75 I einer 25 gew.-%igen wässrigen Ammoniaklösung in den 618,3 kg Lösung) eingerührt. Dann wurde auf 95°C erwärmt und bei dieser Temperatur 30 min nachgerührt. Dann wurden noch 50 I 70°C warmes Wasser ergänzt und auf 80°C erwärmt.

Schließlich wurde die wässrige Suspension sprühgetrocknet (Sprühtrockner der Fa. Niro-Atomizer (Kopenhagen) vom Typ S-5O-N/R, Gaseintrittstemperatur 360°C, Gasaustrittstemperatur 110°C, Gleichstrom). Das Sprühpulver wies einen Partikeldurchmesser von 2 bis 50 µm auf.

75 kg von so erhaltenem Sprühpulver wurden in einem Kneter der Fa. AMK (Aachener Misch- und Knetmaschinen Fabrik vom Typ VIU 160 (Sigma Schaufeln) dosiert und unter Zugaben von 12 I Wasser geknetet (Verweilzeit: 30 min, Temperatur 40 bis 50°C). Danach wurde das Knetgut in einen Extruder (gleicher Extruder wie bei Phase B Herstellung) entleert und mittels des Extruders zu Strängen (Länge 1-10 cm; Durchmesser 6 mm) geformt. Auf einem Bandtrockner wurden die Stränge 1 h bei einer Temperatur von 120°C (Materialguttemperatur) getrocknet.

250 kg von so erhaltenen Strängen wurden im unter "1." beschriebenen Drehrohrofen wie folgt thermisch behandelt (calciniert):
- die thermische Behandlung erfolgte diskontinuierlich mit einer Materialgutmenge von 250 kg;
- der Neigungswinkel des Drehrohres zur Horizontalen betrug ≈ 0°C;
- das Drehrohr rotierte mit 1,5 Umdrehungen/min rechts herum;
- durch das Drehrohr wurde ein Gasstrom von 205 Nm³/h geführt; dieser bestand zu Beginn der thermischen Behandlung aus 180 Nm³/h Luft und 1 x 25 Nm³/h N₂ als Sperrgas; der das Drehrohr verlassende Gasstrom wurde um weitere 1 x 25 Nm³/h N₂ ergänzt; von diesem Gesamtstrom wurden 22 - 25 Vol.-% ins Drehrohr rückgeführt und der Rest ausgelassen; die Auslassmenge wurde durch das Sperrgas und als Restmenge durch Frischluft ergänzt;
- der Gasstrom wurde mit 25°C ins Drehrohr geführt;
- der Druck hinter dem Drehrohrausgangs lag 0,5 mbar unterhalb Außendruck (Normaldruck);
- die Temperatur im Materialgut wurde zunächst innerhalb von 1,5 h linear von 25°C auf 250°C erhöht; dann wurde die Temperatur im Materialgut innerhalb von 2 h linear von 250°C auf 300°C erhöht und diese Temperatur 2 h gehalten; dann wurde die Temperatur im Materialgut innerhalb von 3 h linear von 300°C auf 405°C erhöht und diese Temperatur anschließend 2 h gehalten; dann wurden die Heizzonen abgeschaltet und die Temperatur innerhalb des Materialgutes durch Aktivierung der Schnellkühlung des Drehrohres durch Ansaugen von Luft innerhalb von 1 h auf eine unterhalb von 100°C liegende Temperatur erniedrigt und schließlich auf Umgebungstemperatur abgekühlt.

Die so resultierende pulverförmige Ausgangsmasse 2 wies eine spezifische BET-Oberfläche von 0,6 m²/g und die Zusammensetzung CuSb₂O₆ auf. Das Pulver-Röntgendiagramm des erhaltenen Pulvers zeigte im wesentlichen die Beugungsreflexe von CuSb₂O₆ (Vergleichsspektrum 17-0284 der JCPDS-ICDD-Kartei).

### 4. Herstellung der Ausgangsmasse 3 mit der Stöchiometrie Mo₁₂V_{3,35}W_{1,38}

In einem Rührkessel wurden bei 25°C unter Rühren 900 l Wasser vorgelegt. Anschließend wurden 122,4 kg Ammoniumheptamolybdattetrahydrat (81,5 Gew.-% MoO₃) zugegeben und unter Rühren auf 90°C erhitzt. Dann wurden unter Aufrechterhaltung der 90°C zunächst 22,7 kg Ammoniummetavanadat und schließlich 20,0 kg Ammoniumparawolframatheptahydrat (88,9 Gew.-% WO₃) eingerührt. Durch insgesamt 80-minütiges Rühren bei 90°C wurde eine klare orangefarbene Lösung erhalten. Diese wurde auf 80°C abgekühlt. Dann wurden unter Beibehalt der 80°C zunächst 18,8 kg Essigsäure (≈ 100 gew.-%ig, Eisessig) und dann 24 l 25 gew.-%ige wässrige Ammoniaklösung eingerührt.

Die Lösung blieb klar und orangefarben und wurde mit einem Sprühtrockner der Fa. Niro-Atomizer (Kopenhagen) vom Typ S-50-N/R sprühgetrocknet (Gaseintrittstemperatur: 260°C, Gasaustrittstemperatur: 110°C, Gleichstrom). Das resultierende Sprühpulver bildete die Ausgangsmasse 3 und wies einen Partikeldurchmesser von 2 bis 50 µm auf.

### 5. Herstellung der thermisch zu behandelnden Trockenmasse mit der Stöchiometrie (Mo₁₂V_{3,46}W_{1,39})_{0,87} (CuMo_{0,5}W_{0,5}O₄)_{0,4} (CuSb₂O₆)_{0,4}

In einem Trogkneter der Fa. AMK (Aachener Misch- und Knetmaschinen Fabrik) vom Typ VIU 160 mit zwei Sigmaschaufeln wurden 75 kg Ausgangsmasse 3, 5,2 I Wasser und 6,9 kg Essigsäure (100 Gew.-% Eisessig) vorgelegt und während 22 min geknetet. Anschließend wurden 3,1 kg Ausgangsmasse 1 und 4,7 kg Ausgangsmasse 2 zugegeben und während weiterer 8 min geknetet (T = 40 bis 50°C).

Danach wurde das Knetgut in einen Extruder (gleicher Extruder wie bei Phase B Herstellung) entleert und mittels des Extruders zu Strängen (1 bis 10 cm Länge, 6 mm Durchmesser) geformt. Diese wurden auf einem Bandtrockner während 1 h bei einer Temperatur (Materialguttemperatur) von 120°C getrocknet.

306 kg der getrockneten Stränglinge wurden anschließend im unter "1." beschriebenen Drehrohrofen wie folgt thermisch behandelt:
- die thermische Behandlung erfolgte diskontinuierlich mit einer Materialgutmenge von 306 kg;
- der Neigungswinkel des Drehrohres zur Horizontalen betrug = 0°;
- das Drehrohr rotierte mit 1,5 Umdrehungen/min rechts herum;
- zunächst wurde die Materialguttemperatur innerhalb von 2 h im wesentlichen linear von 25°C auf 100°C erhitzt;
während dieser Zeit wird durch das Drehrohr ein (im wesentlichen) Stickstoffstrom von 205 Nm³/h zugeführt. Im stationären Zustand (nach Verdrängung der ursprünglich enthaltenen Luft) ist dieser wie folgt zusammengesetzt:
110 Nm³/h Grundlast - Stickstoff (20),
25 Nm³/h Sperrgas - Stickstoff (11), und
70 Nm³/h rezirkuliertes Kreisgas (19).

Der Sperrgas-Stickstoff wurde mit einer Temperatur von 25°C zugeführt. Das Gemisch der beiden anderen Stickstoffströme wurde jeweils mit der Temperatur ins Drehrohr geführt, die das Materialgut im Drehrohr jeweils aufwies.
- anschießend wurde die Materialguttemperatur mit einer Heizrate von 0,7°C/min von 100°C auf 320°C aufgeheizt;
bis zum Erreichen einer Materialguttemperatur von 300°C wurde durch das Drehrohr ein Gasstrom von 205 Nm³/h geführt, der wie folgt zusammengesetzt war:
110 Nm³/h bestehend aus Grundlast- Stickstoff (20) und im Drehrohr freigesetzten Gasen,
25 Nm³/h Sperrgas - Stickstoff (11) und
70 Nm³/h rezirkuliertes Kreisgas (19).

Der Sperrgas-Stickstoff wurde mit einer Temperatur von 25°C zugeführt. Das Gemisch der beiden anderen Gasströme wurde jeweils mit der Temperatur ins Drehrohr geführt, die das Materialgut im Drehrohr jeweils aufwies.

Ab Überschreiten der Materialguttemperatur von 160°C bis zum Erreichen einer Materialguttemperatur von 300°C wurden aus dem Materialgut 40 Mol-% der im Verlauf der gesamten thermischen Behandlung des Materialgutes insgesamt freigesetzten Ammoniakmenge M^{A} freigesetzt.
- bei Erreichen der Materialguttemperatur von 320°C wurde der Sauerstoffgehalt des dem Drehrohr zugeführten Gasstromes von 0 Vol.-% auf 1,5 Vol.-% erhöht und über die nachfolgenden 4 h beibehalten.

Gleichzeitig wurde die in den vier das Drehrohr beheizenden Heizzonen herrschende Temperatur um 5°C abgesenkt (auf 325°C) und so während der nachfolgenden 4 h aufrechterhalten.

Die Materialguttemperatur durchlief dabei ein oberhalb von 325°C liegendes Temperaturmaximum, das 340°C nicht überschritt, bevor die Materialguttemperatur wieder auf 325°C sank.

Die Zusammensetzung des durch das Drehrohr geführten Gasstroms von 205 Nm³/h wurde während dieses Zeitraums von 4 h wie folgt geändert:
95 Nm³/h bestehend aus Grundlast - Stickstoff (20) und im Drehrohr freigesetzten Gasen;
25 Nm³/h Sperrgas - Stickstoff (11);
70 Nm³/h rezirkuliertes Kreisgas; und
15 Nm³/h Luft über den Splitter (21).

Der Sperrgas-Stickstoff wurde mit einer Temperatur von 25°C zugeführt.

Das Gemisch der anderen Gasströme wurde jeweils mit der Temperatur ins Drehrohr geführt, die das Materialgut im Drehrohr jeweils aufwies.

Ab Überschreiten der Materialguttemperatur von 300°C bis zum Ablauf der 4 h wurden aus dem Materialgut 55 mol% der im Verlauf der gesamten thermischen Behandlung des Materialguts insgesamt freigesetzten Ammoniakmenge M^{A} freigesetzt (damit waren bis zum Ablauf der 4 h insgesamt 40 Mol-% + 55 Mol-% = 95 Mol% der Ammoniakmenge M^{A} freigesetzt).
- mit Ablauf der4 h wurde die Temperatur des Materialguts mit einer Heizrate von 0,85°C/min innerhalb von etwa 1,5 h auf 400°C erhöht.

Anschließen wurde diese Temperatur 30 min aufrechtgehalten.

Die Zusammensetzung des dem Drehrohr zugeführten Gasstroms von 205 Nm³/h war während dieser Zeit wie folgt:
95 Nm³/h zusammengesetzt aus Grundlast - Stickstoff (20) und im Drehrohr freigesetzten Gasen;
15 Nm³/h Luft (Splitter (21));
25 Nm³/h Sperrgas-Stickstoff (11); und
70 Nm³/h rezirkuliertes Kreisgas.

Der Sperrgas-Stickstoff wurde mit einer Temperatur von 25°C zugeführt. Das Gemisch der anderen Gasströme wurde jeweils mit der Temperatur ins Drehrohr geführt, die das Materialgut im Drehrohr jeweils aufwies.
- durch Verringerung der Temperatur des Materialguts wurde die Calcination beendet; dazu wurden die Heizzonen aus- und die Schnellkühlung des Drehrohres durch Ansaugen von Luft eingeschaltet, und die Materialguttemperatur innerhalb von 2 h auf eine unterhalb von 100°C liegende Temperatur erniedrigt und schließlich auf Umgebungstemperatur abgekühlt;
mit der Abschaltung der Heizzonen wurde die Zusammensetzung des dem Drehrohr zugeführten Gasstroms von 205 Nm³/h auf folgendes Gemisch verändert:
110 Nm³/h zusammengesetzt aus Grundlast - Stickstoff (20) und im Drehrohr freigesetzten Gasen;
0 Nm³/h Luft (Splitter (21));
25 Nm³/h Sperrgas-Stickstoff (11); und
70 Nm³/h rezirkuliertes Kreisgas.

Der Gasstrom wurde dem Drehrohr mit einer Temperatur von 25°C zugeführt.
- während der gesamten thermischen Behandlung lag der Druck (unmittelbar) hinter dem Drehrohrausgang 0,2 mbar unterhalb des Außendrucks.

### 6. Formgebung der Multimetalloxidaktivmasse

Das in "5." erhaltene katalytisch aktive Material wurde mittels einer Biplexquerstromsichtmühle (BQ 500) (Fa. Hosokawa-Alpine Augsburg) zu einem feinteiligen Pulver gemahlen, von dem 50 % der Pulverpartikel ein Sieb der Maschenweite 1 bis 10 µm passierten und dessen Anteil an Partikeln mit einer Längstausdehnung oberhalb von 50 µm weniger als 1 % betrug.

Mittels des gemahlenen Pulvers wurden wie in S1 der EP-B 714700 ringförmige Trägerkörper (7 mm Außendurchmesser, 3 mm Länge, 4 mm Innendurchmesser, Steatit des Typs C220 der Fa. CeramTec mit einer Oberflächenrauhigkeit Rz von 45 µm) beschichtet. Bindemittel war eine wässrige Lösung aus 75 Gew.-% Wasser und 25 Gew.-% Glycerin.

Der Aktivmassenanteil der resultierenden Schalenkatalysatoren wurde jedoch im Unterschied zu vorgenanntem Beispiel S1 zu 20 Gew.-% (bezogen auf das Gesamtgewicht aus Trägerkörper und Aktivmasse) gewählt. Das Verhältnis von Pulver und Bindemittel wurde proportional angepasst.

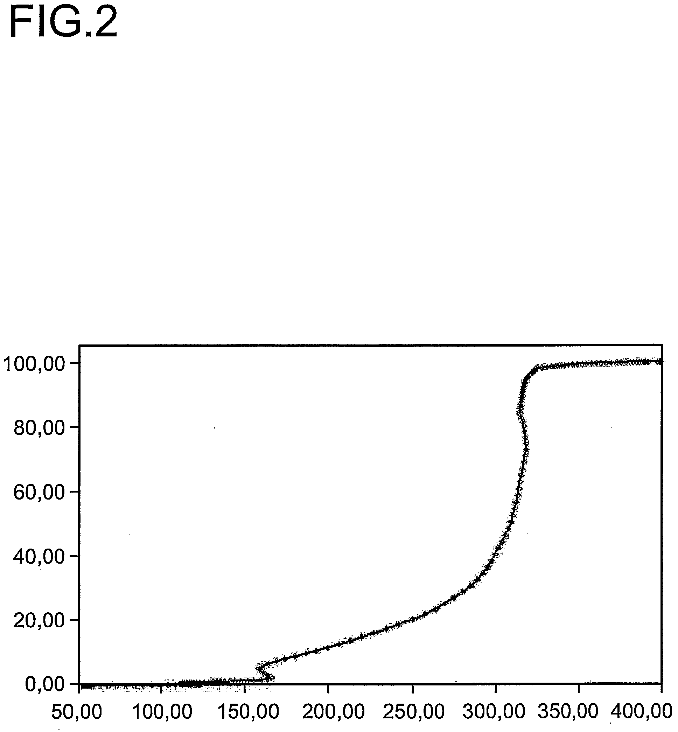

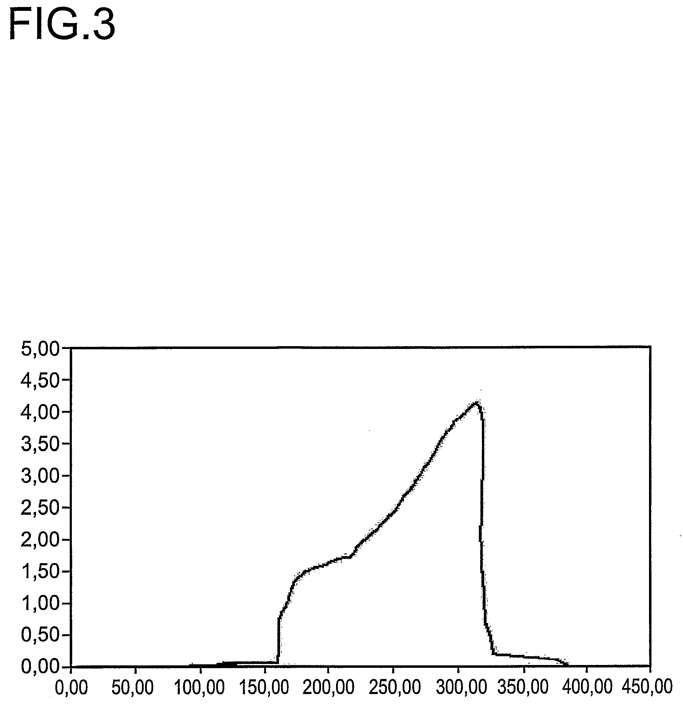

Figur 2 zeigt den prozentualen Anteil von M^{A} als Funktion der Materialguttemperatur in °C. Figur 3 zeigt die Ammoniakkonzentration der Atmosphäre A in Vol.-% über die thermische Behandlung in Abhängigkeit von der Materialguttemperatur in °C.

### B) Durchführung der Partialoxidation

### I. Beschreibung der allgemeinen Verfahrensbedingungen

| | |
|---|---|
| Verwendetes Wärmeaustauschmittel: | Salzschmelze, bestehend aus |
| | 60 Gew.-% Kaliumnitrat und |
| | 40 Gew.-% Natriumnitrit |
| | |
| Material der Kontaktrohre: | ferritischer Stahl |
| | |
| Abmessungen der Kontaktrohre: | 3200 mm Länge; |
| | 25 mm Innendurchmesser; |
| | 30 mm Außendurchmesser |
| | (Wandstärke: 2,5 mm) |
| | |
| Anzahl der Kontaktrohre im Rohrbündel: | 25500 |

Reaktor:
   Zylinderförmiger Behälter eines Durchmessers von 6800 mm; ringförmig angeordnetes Rohrbündel mit einem freien zentralen Raum.
   Durchmesser des zentralen freien Raumes: 1000 mm. Abstand der am weitesten außen liegenden Kontaktrohre zur Behälterwand: 150 mm. Homogene Kontaktrohrverteilung im Rohrbündel (6 äquidistante Nachbarrohre pro Kontaktrohr).
   Kontaktrohrteilung: 38 mm.
   Die Kontaktrohre waren mit ihren Enden in Kontaktrohrböden der Dicke 125 mm abdichtend befestigt und mündeten mit ihren Öffnungen in je eine am oberen bzw. unteren Ende mit dem Behälter verbundenen Haube.
   Zuführung des Wärmeaustauschmittels zum Rohrbündel:
      Das Rohrbündel war durch drei zwischen den Kontaktrohrböden längs derselben aufeinanderfolgend angebrachte Umlenkscheiben (Dicke jeweils 10 mm) in 4 äquidistante (jeweils 730 mm) Längsabschnitte (Zonen) geteilt.

Die unterste und die oberste Umlenkscheibe wies Ringgeometrie auf, wobei der Ringinnendurchmesser 1000 mm betrug und der Ringaußendurchmesser sich bis zur Behälterwand abdichtend erstreckte. Die Kontaktrohre waren an den Umlenkscheiben nicht abdichtend befestigt. Vielmehr waren eine Spaltbreite < 0,5 mm aufweisende Spalte so belassen, dass die Querströmungsgeschwindigkeit der Salzschmelze innerhalb einer Zone möglichst konstant war.

Die mittlere Umlenkscheibe war kreisförmig und erstreckte sich bis zu den am weitesten außen liegenden Kontaktrohren des Rohrbündels.

Die Kreisführung der Salzschmelze wurde durch zwei Salzpumpen bewerkstelligt, von denen jede eine Rohrbündellängshälfte versorgte.

Die Pumpen drückten die Salzschmelze in einen um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floß dann der Vorgabe der Umlenkbleche folgend in der Abfolge
- von außen nach innen,
- von innen nach außen,
- von außen nach innen,
- von innen nach außen,
im wesentlichen mäanderförmig, über den Behälter betrachtet, von unten nach oben. Durch im obersten Längsabschnitt um den Behälterumfang angebrachte Fenster sammelte sich die Salzschmelze in einem oberen um den Reaktormantel angebrachten Ringkanal und wurde nach Abkühlung auf die ursprüngliche Eintrittstemperatur durch die Pumpen wieder in den unteren Ringkanal gedrückt.

Die Zusammensetzung des Reaktionsgasausgangsgemisches lag über die Betriebszeit in folgendem Raster:
4 bis 6 Vol.-% Acrolein,
5 bis 8 Vol.-% O₂,
1,2 bis 2,5 Vol.-% COₓ,
6 bis 10 Vol.-% H₂O und
wenigstens 75 Vol.-% N₂.

| | |
|---|---|
| Reaktorbeschickung: | Salzschmelze und Reaktionsgasgemisch wurden über den Reaktor betrachtet im Gegenstrom geführt. Die Salzschmelze trat unten ein, das Reaktionsgasgemisch oben. |
| | |
| | Die Eintrittstemperatur der Salzschmelze betrug am Anfang (nach beendeter Formierung des Katalysatorfestbetts) ca. 265°C. Die zugehörige Austrittstemperatur der Salzschmelze lag am Anfang bei ca. 267°C. |
| | |
| | Die Pumpleistung betrug 6200 m³ Salzschmelze/h. |
| | |
| | Das Reaktionsgasausgangsgemisch wurde dem Reaktor |
| | mit einer Temperatur von 240°C zugeführt. |
| | |
| Acroleinlast des | |
| Katalysatorfestbetts: | 95 bis 110 Nl/l•h |

Die Kontaktrohrbechickung mit Katalysatorfestbett

| | |
|---|---|
| (von oben nach unten) war: | Zone A: |
| | 20 cm Vorschüttung aus Steatit-Ringen der Geometrie 7 mm x 7 mm x 4 mm (Außendurchmesser x Länge x Innendurchmesser). |
| | |
| | Zone B: |
| | 100 cm Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% des ringförmigen (ca. 7 mm x 3 mm x 4 mm) hergestellten Schalenkatalysators. |
| | |
| | Zone C: |
| | 200 cm Katalysatorbeschickung mit dem ringförmigen (ca. 7 mm x 3 mm x 4 mm) hergestellten Schalenkatalysator. |

Die Thermorohre (ihre Anzahl belief sich auf 10, die im zentralen Bereich des Rohrbündels gleichmäßig verteilt waren) waren wie folgt gestaltet und beschickt:
(mit ihnen wurde die Heißpunkttemperatur ermittelt; sie ist ein arithmetischer Mittelwert aus voneinander unabhängigen Messungen in den 10 Thermorohren)
   Jeder der 10 Thermorohre wies eine zentrale Thermohülle mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte).
   Wenigstens 13 und höchstens 30 der jeweils 40 Temperaturmessstellen befand sich im Bereich des ersten Meters des aktiven Abschnitts des Katalysatorfestbetts (in Strömungsrichtung des Reaktionsgasgemischs).
   Der Innendurchmesser eines Thermorohres betrug 27 mm. Die Wanddicke und das Rohrmaterial war wie bei den Arbeitsrohren beschaffen.
   Der Außendurchmesser der Thermohülse betrug 4 mm.
   Ein Thermorohr wurde mit dem hergestellten ringförmigen Schalenkatalysator befüllt. Zusätzlich wurde in das Thermorohr kugelförmiger Schalenkatalysator beigefüllt (gleiche Aktivmasse wie der ringförmige Schalenkatalysator, der Durchmesser der Steatit C220 (CeramTec) Trägerkugeln betrug 2-3 mm; der Aktivmassenanteil betrug 20 Gew.-%, die Herstellung erfolgte wie bei dem ringförmigen Schalenkatalysator beschrieben, Bindemittel war jedoch eine entsprechende Menge Wasser).
   Die Beifüllung des kugelförmigen Schalenkatalysators erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu 5 bis 20 Gew.-% an kugelförmigem Schalenkatalysator erforderlich). Gleichzeitig war die jeweilige Gesamtfüllhöhe von Aktiv- und Inertabschnitten in den Arbeitsund Thermorohren gleich bemessen und das Verhältnis von im Rohr enthaltener Gesamtmenge an Aktivmasse zu Wärmeübergangsfläche des Rohres bei Arbeits- und Thermorohren auf denselben Wert eingestellt.

### II. Langzeitbetrieb (Ergebnisse)

Das Umsatzziel für das bei einmaligem Durchgang des Reaktionsgasgemischs durch das Katalysatorfestbett umzusetzende Acrolein wurde auf 99,3 mol-% festgelegt.

Durch sukzessives Erhöhen der Eintrittstemperatur der Salzschmelze konnte dieser Umsatzwert bei kontinuierlicher Durchführung des Verfahrens über die Zeit aufrechterhalten werden. Einmal je Kalendermonat wurde die Partialoxidation unterbrochen (die Erhöhung der Eintrittstemperatur der Salzschmelze bis zur monatlichen Unterbrechung betrug stets ≥ 0,3°C und ≤ 4°C), die zuletzt angewandte Eintrittstemperatur der Salzschmelze beibehalten und für eine Zeitdauer t_{G} von 24 h bis 48 h ein Gasgemisch G mit einer Belastung des Katalysatorfestbetts von 30 Nl/l•h durch das Katalysatorfestbett geführt. Der Sauerstoffgehalt des Gasgemischs G wurde im Verlauf der Zeitdauer t_{G} von ca. 2 Vol.% auf 6 Vol.-% erhöht. Der CO- und der CO₂-Gehalt des Gasgemischs G wurde im Verlauf der Zeitdauer t_{G} von Werten ≤ 1 Vol.-% (CO) bzw. ≤ 4 Vol.-% (CO₂) auf 0 Vol.-% erniedrigt, der Wasserdampfgehalt lag bei ≤ 6 Vol.-%, der Gehalt an Molybdänoxidhydrat lag bei ≤ 1 Gew.ppm und die Restmenge des Gasgemischs G bestand im wesentlichen aus Stickstoff.

Dann wurde die Partialoxidation fortgesetzt und die Eintrittstemperatur der Salzschmelze so eingestellt, dass das Umsatzziel weiter erreicht wurde.

Die Eintrittstemperatur der Salzschmelze und die Heißpunkttemperatur sowie die Selektivität S^{AA+} der Acrylsäurebildung entwickelten sich dabei wie folgt:

| | | |
|---|---|---|
| Anfang: | Salzschmelzeeintrittstemperatur | = 265°C |
| | Heißpunkttemperatur | = 290°C |
| | S^{AA} | = 94,3 mol-% |
| Nach 1 Jahr | | |
| Betriebsdauer: | Salzschmelzeeintrittstemperatur | = 276°C |
| | Heißpunkttemperatur | = 297°C |
| | S^{AA} | = 94,2 mol-% |
| Nach 2 Jahren | | |
| Betriebsdauer: | Salzschmelzeeintrittstemperatur | = 282°C |
| | Heißpunkttemperatur | = 303°C |
| | S^{AA} | = 94,3 mol-% |
| Nach 3 Jahren | | |
| Betriebsdauer: | Salzschmelzeeintrittstemperatur | = 299°C |
| | Heißpunkttemperatur | = 312°C |
| S^{AA} | | = 94,4 mol-% |

Innerhalb der 3 Jahre Betriebsdauer wanderte der Ort der Heißpunkttemperatur etwa um ca. 25 cm in Richtung der Eintrittsstelle des Reaktionsgasgemischs.

In einem zweiten baugleichen und identisch beschickten sowie betriebenen Rohrbündelreaktor wurde die Partialoxidation in entsprechender Weise durchgeführt und dabei folgende Ergebnisse erzielt:

| | | |
|---|---|---|
| Anfang: | Salzschmelzeeintrittstemperatur | = 266°C |
| | Heißpunkttemperatur | = 292°C |
| | S^{AA} | = 94,6 mol-% |
| Nach 1 Jahr | | |
| Betriebsdauer: | Salzschmelzeeintrittstemperatur | = 274°C |
| | Heißpunkttemperatur | = 299°C |
| | S^{AA} | = 94,6 mol-% |
| Nach 2 Jahren | | |
| Betriebsdauer: | Salzschmelzeeintrittstemperatur | = 283°C |
| | Heißpunkttemperatur | = 305°C |
| | S^{AA} | = 94,7 mol-% |
| Nach 3 Jahren | | |
| Betriebsdauer: | Salzschmelzeeintrittstemperatur | = 299°C |
| | Heißpunkttemperatur | = 311 °C |
| | S^{AA} | = 94,7 mol-% |

Die Wanderung des Ortes der Heißpunkttemperatur war wie oben.

Die Temperaturangaben (abgesehen für den Anfang) beziehen sich in allen Fällen jeweils auf den Zeitpunkt, der kurz vor der Unterbrechung der Partialoxidation und der Behandlung des Katalysatorfestbetts mit dem Gasgemisch G lag.

## Patentansprüche

1. Ein Verfahren zum Langzeitbetrieb einer heterogen katalysierten Gasphasenpartialoxidation von Acrolein zu Acrylsäure, bei dem man ein Acrolein, molekularen Sauerstoff und wenigstens ein inertes Verdünnungsgas enthaltendes Reaktionsgasausgangsgemisch durch ein bei erhöhter Temperatur befindliches Katalysatorfestbett führt, dessen Katalysatoren so beschaffen sind, dass ihre Aktivmasse wenigstens ein Multimetalloxid ist, das die Elemente Mo und V enthält und, bei dem man, um der Deaktivierung des Katalysatorfestbetts entgegenzuwirken, die Temperatur des Katalysatorfestbetts über die Zeit erhöht,
**dadurch gekennzeichnet,**
**dass** man, bevor die Temperaturerhöhung des Katalysatorfestbetts dauerhaft ≥ 10°C beträgt, die Gasphasenpartialoxidation wenigstens einmal unterbricht, und bei einer Temperatur des Katalysatorfestbetts von 200 bis 450°C ein an Acrolein freies, molekularen Sauerstoff, Inertgas und gegebenenfalls Wasserdampf sowie gegebenenfalls CO enthaltendes, oxidierend wirkendes Gasgemisch G durch das Katalysatorfestbett führt.

2. Verfahren nach Anspruch 1, **dadurch gekennzeichnet, dass** man, bevor die Temperaturerhöhung des Katalysatorfestbetts dauerhaft ≥ 8°C beträgt, die Gasphasenpartialoxidation wenigstens einmal unterbricht und das Gasgemisch G durch das Katalysatorfestbett führt.

3. Verfahren nach Anspruch 1, **dadurch gekennzeichnet, dass** man, bevor die Temperaturerhöhung des Katalysatorfestbetts dauerhaft ≥ 4°C beträgt, die Gasphasenpartialoxidation wenigstens einmal unterbricht und das Gasgemisch G durch das Katalysatorfestbett führt.

4. Verfahren nach Anspruch 1, **dadurch gekennzeichnet, dass** man, bevor die Temperaturerhöhung des Katalysatorfestbetts dauerhaft ≥ 2°C beträgt, die Gasphasenpartialoxidation wenigstens einmal unterbricht und das Gasgemisch G durch das Katalysatorfestbett führt.

5. Verfahren nach einem der Ansprüche 1 bis 4, **dadurch gekennzeichnet, dass** die Zeitdauer, während der das Gasgemisch G durch das Katalysatorfestbett geführt wird, 2 h bis 120 h beträgt.

6. Verfahren nach einem der Ansprüche 1 bis 5, **dadurch gekennzeichnet, dass** das Gasgemisch G, das durch das Katalysatorfestbett geführt wird, wenigstens 2 Vol.-% Sauerstoff enthält.

7. Verfahren nach einem der Ansprüche 1 bis 5, **dadurch gekennzeichnet, dass** das Gasgemisch G, das durch das Katalysatorfestbett geführt wird, wenigstens 3 Vol.-% Sauerstoff enthält.

8. Verfahren nach einem der Ansprüche 1 bis 5, **dadurch gekennzeichnet, dass** das Gasgemisch G, das durch das Katalysatorfestbett geführt wird, 1 bis 8 Vol.-% Sauerstoff, 0 bis 3 Vol.-% CO, 0 bis 5 Vol.-% CO₂, 0 bis 25 Vol.-% H₂O und wenigstens 55 Vol.% N₂ enthält.

9. Verfahren nach einem der Ansprüche 1 bis 8, **dadurch gekennzeichnet, dass** die Aktivmasse der Katalysatoren wenigstens ein Multimetalloxid der allgemeinen Formel I,
Mo₁₂VₐX¹_{b}X²_{c}X³_{d}X⁴ₑX⁵_{f}X⁶_{g}Oₙ (I),
in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in I bestimmt wird,
ist.

10. Verfahren nach einem der Ansprüche 1 bis 9, **dadurch gekennzeichnet, dass** es in einem Rohrbündelreaktor durchgeführt wird.

11. Verfahren nach einem der Ansprüche 1 bis 10, **dadurch gekennzeichnet, dass** die Erhöhung der Temperatur des Katalysatorfestbetts über die Zeit so durchgeführt wird, dass der Acroleingehalt im Produktgasgemisch 1500 Gew.ppm nicht übersteigt.

12. Verfahren nach einem der Ansprüche 1 bis 11, **dadurch gekennzeichnet, dass** die Belastung des Katalysatorfestbetts mit Acrolein ≥ 90 Nl/l•h beträgt.

13. Verfahren nach einem der Ansprüche 1 bis 12, **dadurch gekennzeichnet, dass** die Belastung des Katalysatorfestbetts mit Acrolein ≥ 130 Nl/l•h beträgt.

14. Verfahren nach einem der Ansprüche 1 bis 13, **dadurch gekennzeichnet, dass** der CO-Gehalt des Gasgemischs G innerhalb der Zeitdauer t_{G}, während der Gasgemisch G durch das Katalysatorfestbett geführt wird, von einem von 0 verschiedenen Anfangswert abfällt.

15. Verfahren nach einem der Ansprüche 1 bis 14, **dadurch gekennzeichnet, dass** das Gasgemisch G > 0 bis ≤ 20 Gew.ppm einer gasförmigen Mo-enthaltenden Verbindung aufweist.

16. Verfahren nach einem der Ansprüche 1 bis 15, **dadurch gekennzeichnet, dass** der Sauerstoffgehalt des Gasgemischs G innerhalb der Zeitdauer t_{G} während der Gasgemisch G durch das Katalysatorfestbett geführt wird, von einem niederen Anfangswert auf einen höheren Endwert erhöht wird.

17. Verfahren nach einem der Ansprüche 1 bis 16, **dadurch gekennzeichnet, dass** die Erhöhung der Temperatur des Katalysatorfestbetts über die Zeit so durchgeführt wird, dass der Umsatz des Acroleins bei einmaligem Durchgang des Reaktionsgasgemischs durch das Katalysatorfestbett 94 mol-% nicht unterschreitet.

18. Verfahren nach einem der Ansprüche 1 bis 17, **dadurch gekennzeichnet, dass** das Reaktionsgasausgangsgemisch 6 bis 15 Vol.-% Acrolein enthält.

## Claims

1. A process for the long-term operation of a heterogeneously catalyzed gas phase partial oxidation of acrolein to acrylic acid, by conducting a starting reaction gas mixture which comprises acrolein, molecular oxygen and at least one inert diluent gas through a fixed catalyst bed at elevated temperature whose catalysts are such that their active composition is at least one multimetal oxide which contains the elements Mo and V, and by, in order to counteract the deactivation of the fixed catalyst bed, increasing the temperature of the fixed catalyst bed over time,
which comprises
interrupting the gas phase partial oxidation at least once before the temperature increase of the fixed catalyst bed is permanently ≥ 10°C, and, at a temperature of the fixed catalyst bed of from 200 to 450°C, conducting an acrolein-free, oxidizing gas mixture G comprising molecular oxygen, inert gas and optionally steam and also optionally CO through the fixed catalyst bed.

2. A process as claimed in claim 1, wherein the gas phase partial oxidation is interrupted at least once before the temperature increase of the fixed catalyst bed is permanently ≥ 8°C and the gas mixture G is conducted through the fixed catalyst bed.

3. A process as claimed in claim 1, wherein the gas phase partial oxidation is interrupted at least once before the temperature increase of the fixed catalyst bed is permanently ≥ 4°C and the gas mixture G is conducted through the fixed catalyst bed.

4. A process as claimed in claim 1, wherein the gas phase partial oxidation is interrupted at least once before the temperature increase of the fixed catalyst bed is permanently ≥ 2°C and the gas mixture G is conducted through the fixed catalyst bed.

5. A process as claimed in any of claims 1 to 4, wherein the duration over which the gas mixture G is conducted through the fixed catalyst bed is from 2 h to 120 h.

6. A process as claimed in any of claims 1 to 5, wherein the gas mixture G which is conducted through the fixed catalyst bed contains at least 2% by volume of oxygen.

7. A process as claimed in any of claims 1 to 5, wherein the gas mixture G which is conducted through the fixed catalyst bed contains at least 3% by volume of oxygen.

8. A process as claimed in any of claims 1 to 5, wherein the gas mixture G which is conducted through the fixed catalyst bed contains from 1 to 8% by volume of oxygen, from 0 to 3% by volume of CO, from 0 to 5% by volume of CO₂, from 0 to 25% by volume of H₂O and at least 55% by volume of N₂.

9. A process as claimed in any of claims 1 to 8, wherein the active composition of the catalysts is at least one multimetal oxide of the general formula I
MO₁₂VₐX¹_{b}X²_{c}X³_{d}X⁴ₑX⁵_{f}X⁶_{g}Oₙ (I),
in which the variables are each defined as follows:
X¹ =W, Nb, Ta, Cr and/or Ce,
X² =Cu, Ni, Co, Fe, Mn and/or Zn,
X³ =Sb and/or Bi,
X⁴ =one or more alkali metals,
X⁵ =one or more alkaline earth metals,
X⁶ =Si, Al, Ti and/or Zr,
a = from 1 to 6,
b = from 0.2 to 4,
c = from 0.5 to 18,
d = from 0 to 40,
e = from 0 to 2,
f = from 0 to 4,
g = from 0 to 40 and
n = a number which is determined by the valency and frequency of the elements in I other than oxygen.

10. A process as claimed in any of claims 1 to 9, which is carried out in a tube bundle reactor.

11. A process as claimed in any of claims 1 to 10, wherein the increase in the temperature of the fixed catalyst bed over time is carried out in such a way that the acrolein content in the product gas mixture does not exceed 1500 ppm by weight.

12. A process as claimed in any of claims 1 to 11, wherein the acrolein hourly space velocity on the fixed catalyst bed is ≥ 90 1 (STP)/l•h.

13. A process as claimed in any of claims 1 to 12, wherein the acrolein hourly space velocity on the fixed catalyst bed is ≥ 130 l (STP)/l•h.

14. A process as claimed in any of claims 1 to 13, wherein the CO content of the gas mixture G falls from a nonzero starting value within the period t_{G} over which the gas mixture G is conducted through the fixed catalyst bed.

15. A process as claimed in any of claims 1 to 14, wherein the gas mixture G has from > 0 to ≤ 20 ppm by weight of a gaseous Mo-containing compound.

16. A process as claimed in any of claims 1 to 15, wherein the oxygen content of the gas mixture G is increased from a lower starting value to a higher end value within the period t_{G} over which the gas mixture G is conducted through the fixed catalyst bed.

17. A process as claimed in any of claims 1 to 16, wherein the increase in the temperature of the fixed catalyst bed over time is carried out in such a way that the conversion of the acrolein on single pass of the reaction gas mixture through the fixed catalyst bed does not go below 94 mol%.

18. A process as claimed in any of claims 1 to 17, wherein the starting reaction gas mixture contains from 6 to 15% by volume of acrolein.

## Revendications

1. Procédé d'exploitation à long terme d'une oxydation partielle en phase gazeuse sous catalyse hétérogène d'acroléine en acide acrylique, selon lequel un mélange réactionnel gazeux initial contenant de l'acroléine, de l'oxygène moléculaire et au moins un gaz diluant inerte traverse un lit catalytique fixe qui se trouve à température élevée, dont les catalyseurs sont tels que leur masse active soit au moins un oxyde de plusieurs métaux qui contient les éléments Mo et V, et selon lequel la température du lit catalytique fixe est augmentée avec le temps pour contrer la désactivation du lit catalytique fixe,
**caractérisé en ce que**
l'oxydation partielle en phase gazeuse est interrompue au moins une fois avant que l'élévation de température du lit catalytique fixe ne soit durablement ≥ 10 °C et un mélange gazeux oxydant G exempt d'acroléine, contenant de l'oxygène moléculaire, un gaz inerte et éventuellement de la vapeur d'eau, ainsi qu'éventuellement CO, traverse le lit catalytique fixe à une température du lit catalytique fixe de 200 à 450 °C.

2. Procédé selon la revendication 1, **caractérisé en ce que** l'oxydation partielle en phase gazeuse est interrompue au moins une fois avant que l'élévation de température du lit catalytique fixe ne soit durablement ≥ 8 °C, et le mélange gazeux G traverse le lit catalytique fixe.

3. Procédé selon la revendication 1, **caractérisé en ce que** l'oxydation partielle en phase gazeuse est interrompue au moins une fois avant que l'élévation de température du lit catalytique fixe ne soit durablement ≥ 4 °C, et le mélange gazeux G traverse le lit catalytique fixe.

4. Procédé selon la revendication 1, **caractérisé en ce que** l'oxydation partielle en phase gazeuse est interrompue au moins une fois avant que l'élévation de température du lit catalytique fixe ne soit durablement ≥ 2 °C, et le mélange gazeux G traverse le lit catalytique fixe.

5. Procédé selon l'une quelconque des revendications 1 à 4, **caractérisé en ce que** la durée pendant laquelle le mélange gazeux G traverse le lit catalytique fixe est de 2 h à 120 h.

6. Procédé selon l'une quelconque des revendications 1 à 5, **caractérisé en ce que** le mélange gazeux G qui traverse le lit catalytique fixe contient au moins 2 % en volume d'oxygène.

7. Procédé selon l'une quelconque des revendications 1 à 5, **caractérisé en ce que** le mélange gazeux G qui traverse le lit catalytique fixe contient au moins 3 % en volume d'oxygène.

8. Procédé selon l'une quelconque des revendications 1 à 5, **caractérisé en ce que** le mélange gazeux G qui traverse le lit catalytique fixe contient 1 à 8 % en volume d'oxygène, 0 à 3 % en volume de CO, 0 à 5 % en volume de CO₂, 0 à 25 % en volume d'H₂O et au moins 55 % en volume de N₂.

9. Procédé selon l'une quelconque des revendications 1 à 8, **caractérisé en ce que** la masse active des catalyseurs est au moins un oxyde de plusieurs métaux de formule générale I
Mo₁₂VₐX¹_{b}X²_{c}X³_{d}X⁴ₑX⁵_{f}X⁶_{g}Oₙ (I)
dans laquelle les variables ont la signification suivante:
X¹ = W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40, et
n = un nombre déterminé par la valence et la fréquence des éléments différents de l'oxygène dans I.

10. Procédé selon l'une quelconque des revendications 1 à 9, **caractérisé en ce qu'**il est réalisé dans un réacteur à faisceau de tubes.

11. Procédé selon l'une quelconque des revendications 1 à 10, **caractérisé en ce que** l'élévation de la température du lit catalytique fixe avec le temps est réalisée de sorte que la teneur en acroléine du mélange gazeux de produits ne dépasse pas 1 500 ppm en poids.

12. Procédé selon l'une quelconque des revendications 1 à 11, **caractérisé en ce que** le chargement du lit catalytique fixe avec de l'acroléine est ≥ 90 Nl/l·h.

13. Procédé selon l'une quelconque des revendications 1 à 12, **caractérisé en ce que** le chargement du lit catalytique fixe avec de l'acroléine est ≥ 130 Nl/l·h.

14. Procédé selon l'une quelconque des revendications 1 à 13, **caractérisé en ce que** la teneur en CO du mélange gazeux G chute à partir d'une valeur initiale différente de 0 pendant la durée t_{G} pendant laquelle le mélange gazeux G traverse le lit catalytique fixe.

15. Procédé selon l'une quelconque des revendications 1 à 14, **caractérisé en ce que** le mélange gazeux G comprend de > 0 à ≤ 20 ppm en poids d'un composé gazeux contenant Mo.

16. Procédé selon l'une quelconque des revendications 1 à 15, **caractérisé en ce que** la teneur en oxygène du mélange gazeux G augmente d'une valeur initiale plus faible à une valeur finale plus élevée pendant la durée te pendant laquelle le mélange gazeux G traverse le lit catalytique fixe.

17. Procédé selon l'une quelconque des revendications 1 à 16, **caractérisé en ce que** l'élévation de la température du lit catalytique fixe avec le temps est réalisée de sorte que la conversion de l'acroléine lors d'un passage unique du mélange réactionnel gazeux dans le lit catalytique fixe ne soit pas inférieure à 94 % en moles.

18. Procédé selon l'une quelconque des revendications 1 à 17, **caractérisé en ce que** le mélange réactionnel gazeux initial contient 6 à 15 % en volume d'acroléine.
